# EUROPEAN PATENT APPLICATION

(11) **EP 4 718 465 A1**
(43) Date of publication of application: **01.04.2026**
(21) Application number: 25200742.2
(22) Date of filing: 08.09.2025
(51) Int. Cl.: G16H 20/40, G06F 3/02, G06F 18/10, G06T 7/00, A61B 5/00, G16H 30/40, G16H 40/60, G16H 50/20, G16H 50/70, A61B 1/06, A61B 1/24, A61B 1/04, G06V 10/82, G06V 10/98, G06V 10/50, G06V 10/764, A61B 1/00, G06V 10/26, G06V 10/143

(54) **METHOD AND SYSTEM FOR DETERMINING A CONFIDENCE SCORE OF A DENTAL WORKFLOW**

(30) Priority: 30.09.2024 EP 24203437
(71) Applicant: 3Shape A/S, 1060 Copenhagen K (DK)
(72) Inventor: WESTERGAARD, Philip Grabow, 1060 Copenhagen K (DK); VANNAHME, Christoph, 1060 Copenhagen K (DK); PATIL, Sonal Sharad, 1060 Copenhagen K (DK); SPIETZ, Henrik, 1060 Copenhagen K (DK)
(74) Representative: Zacco Denmark A/S

(57) **Abstract**

A computer-implemented method for determining a confidence score (607) of a digital dental workflow (100) is disclosed. The method comprises receiving scan data representative of a dental situation, wherein the scan data comprises color images and near-infrared images. The method further comprises determining a quality measure (106) of the received scan data. Further, the method comprises generating enhanced diagnostic images based on at least the near-infrared images of the scan data and determining a quality measure of the enhanced diagnostic images. The method further comprises identifying a dental condition in the dental situation based on the enhanced diagnostic images and determining a probability value (108) representing a likelihood that the dental condition is identified correctly. The method further comprises determining the confidence score (607) of the digital dental workflow (100) based on the quality measure (106) of the received scan data, the quality measure of the enhanced diagnostic images and the probability value (108).

## Description

### Technical field

The disclosure relates to a computer-implemented method and system for determining a confidence score of a digital dental workflow. In particular, the confidence score of the digital dental workflow is determined based on various metrics associated with different steps of the digital dental workflow such as a quality measure of the received scan data, a quality measure of a generated digital 3D model, a quality measure of enhanced diagnostic images used for identifying a dental condition, and/or a probability value representing likelihood that a dental condition is identified correctly.

### Background

Development of intraoral scanning techniques has been instrumental in the transition to modern digital dentistry. Use of intraoral 3D scanners allows dental practitioners to accurately and quickly capture dental situation of a patient, which may then be visualized on a display device as a digital three-dimensional (3D) model. Obtained digital 3D model may thus serve as a digital impression of teeth, offering numerous advantages over a classical physical impression of teeth.

In a diagnostic digital dental workflow, the digital 3D model and/or the obtained scan data may be analysed for presence of various dental conditions. However, the end result of identifying a certain dental condition is highly dependent on various stages of the digital dental workflow. For example, the scan data representing the dental situation may itself be of varying quality. Furthermore, the quality of the digital 3D model generated based on the scan data may affect the outcome of the workflow. Moreover, the algorithm for detecting the dental condition may be more or less robust to input data and may output results which are not always reliable.

There is a need to develop methods and systems to determine the confidence level of the overall digital dental workflow, its various intermediate stages and the inter-dependencies of the intermediate stages on the end result.

The present disclosure investigates how to assess the various stages of the digital dental workflow to generate the confidence score of the digital dental workflow.

### Summary

In an embodiment, a computer-implemented method for determining a confidence score of a digital dental workflow is disclosed, the method comprising:
- receiving scan data representative of a dental situation,
- determining a quality measure of the received scan data,
- generating a digital 3D model of the dental situation based on the received scan data,
- determining a quality measure of the generated digital 3D model,
- identifying a dental condition on the digital 3D model and determining a probability value that may represent a likelihood that the dental condition is identified correctly,
- determining a confidence score of the digital dental workflow based on the quality measure of the received scan data, the quality measure of the generated digital 3D model and/or the probability value,
- displaying the digital 3D model with the dental condition, and
- displaying the confidence score of the digital dental workflow.

In another embodiment, a computer-implemented method for determining the confidence score of the digital dental workflow is disclosed, the method comprising:
- receiving scan data representative of the dental situation,
- determining the quality measure of the received scan data,
- generating the digital 3D model of the dental situation based on the received scan data,
- determining the quality measure of the generated digital 3D model,
- identifying, on the digital 3D model and based on the scan data, the dental condition and determining the probability value that may represent the likelihood that the dental condition is identified correctly,
- determining the confidence score of the digital dental workflow based on the quality measure of the received scan data, the quality measure of the generated digital 3D model and/or the probability value,
- displaying the digital 3D model with the dental condition, and
- displaying the confidence score of the digital dental workflow.

In yet another embodiment, a computer-implemented method for determining the confidence score of the digital dental workflow is disclosed, the method comprising:
- receiving scan data representative of the dental situation,
- determining the quality measure of the received scan data,
- generating the digital 3D model of the dental situation based on the received scan data,
- identifying the dental condition based on the scan data, and determining the probability value that may represent the likelihood that the dental condition is identified correctly,
- determining the confidence score of the digital dental workflow based on the quality measure of the received scan data, and/or the probability value,
- displaying the digital 3D model with the dental condition, and
- displaying the confidence score of the digital dental workflow.

In a further embodiment, a computer-implemented method for determining the confidence score of the digital dental workflow is disclosed, the method comprising:
- receiving scan data representative of the dental situation, wherein the scan data comprises color images and near-infrared images,
- determining the quality measure of the received scan data,
- generating enhanced diagnostic images based on at least the near-infrared images of the scan data,
- determining a quality measure of the enhanced diagnostic images,
- identifying the dental condition in the dental situation based on the enhanced diagnostic images,
- determining the probability value that may represent the likelihood that the dental condition is identified correctly,
- determining the confidence score of the digital dental workflow based on the quality measure of the received scan data, the quality measure of the enhanced diagnostic images and the probability value.

Expression "3D" throughout the present disclosure refers to the term "three-dimensional". Similarly, term "2D" refers to the term "two-dimensional". Term "digital 3D model" refers to a digital, three-dimensional, computer-generated representation of the patient's dental situation.

Such digital 3D model may accurately correspond to the actual dental situation. That means that dental objects like teeth, teeth surfaces, palate, restorations and/or gingiva on the digital 3D model may correspond to those of the dental situation.

The digital 3D model may be constructed by a processor, based on received scan data or at least part of the received scan data because the scan data may comprise, besides data relevant to generating the digital 3D model, other type of data such as data relevant for identifying the dental condition. In an intraoral scanning process, an intraoral 3D scanner may be used to scan the patient's dental situation comprising teeth and gingiva and thereby acquire the scan data. The intraoral 3D scanner throughout the disclosure is also referred to as the intraoral scanner. The digital 3D model can be stored in a memory of a computer system, for example in a Standard Triangle Language (STL) format or in any other format for displaying or printing 3D objects.

The method according to an embodiment may comprise receiving, for example by the processor, scan data representative of the dental situation. The scan data may comprise 2D image data such as color images, fluorescence images and/or near-infrared images. In an example the color images may be used to generate the digital 3D model as the color images may comprise geometric data required for the digital 3D model buildup. The color images, the fluorescence images and/or the near-infrared images may also be referred to as raw scan data. As will be discussed further in the disclosure, this raw scan data, for example the near-infrared images, may be processed to improve their diagnostic usability.

The fluorescence images and/or the near-infrared images may be referred to as diagnostic image data and may be used for identifying the dental condition. It may be noted that the color images may also carry diagnostic information and may be utilized in identifying of the dental condition. Received fluorescence images and/or near-infrared images may be further processed into enhanced diagnostic images to improve the diagnostic capabilities of the herein disclosed methods and systems.

The method may further comprise determining the quality measure of the received scan data. This step is significant as the quality of scan data may affect down-stream steps of the digital dental workflow. Correctly identifying the dental condition in the dental situation may depend on the quality measure of the received scan data.

The digital dental workflow may be a diagnostic workflow in which the dental condition may be identified in the dental situation of the patient. The digital dental workflow may comprise multiple steps such as receiving scan data of the dental situation, processing of the scan data in order to make it suitable for diagnostic algorithms, generating the digital 3D model based on the received scan data and/or identifying the dental condition.

The quality measure of the received scan data may be displayed, for example on a display device. This information may be advantageous for a user to have because having a timely indication of a bad quality of the received scan data may prompt the user to re-acquire the scan data to improve this quality measure. If this re-acquiring is done in the early stage of the dental workflow, then time can be saved both for the dentist and the patient. By having a timely indication of a good quality of the received scan data the user may be confident that the scanning process was performed successfully and that no re-acquiring of scan data is needed.

Thus, the quality measure of the received scan data may be determined simultaneously during receiving of the scan data. This is advantageous because the quality measure of the received scan data may be displayed to the user as it is being determined, allowing the user to make corrections in the intraoral scanning process.

The method may further comprise generating the digital 3D model based on the received scan data or based on at least a part of the scan data, for example based on the color images of the scan data. The color images may comprise geometric data required for generation of the digital 3D model. In an example, the geometric data may relate to an overlap between a series of color images that are joined together to create individual sub-scans (smaller 3D parts of the digital 3D model). The individual sub-scans may then be fused together to form the digital 3D model.

The digital 3D model may be generated by the processor. The digital 3D model may usually be displayed on the display device in form of a 3D mesh, representing surfaces of teeth and gingival tissue of the dental situation. The 3D mesh may be comprised of individual facets, for example triangular facets while each facet may comprise, for example, three mutually connected vertices. Alternatively, the digital 3D model may be displayed as a point cloud comprising points, a graph comprising nodes and edges, a volumetric representation comprising voxels, or any other suitable 3D representation form.

The method may further comprise determining the quality measure of the generated digital 3D model. The generated digital 3D model may, in some cases, comprise missing information, for example because the scan data was acquired by improperly scanning the dental situation where some parts of the dental situation are not scanned. Such scan data may be referred to as incomplete scan data. The resulting digital 3D model may thus comprise empty spaces, referred to as "holes", where 3D information is missing. Additionally or alternatively, the digital 3D model may comprise some surfaces where color information and/or fluorescence information is missing. This type of missing information may influence how efficient the digital dental workflow is because, in some examples, the digital 3D model may be directly processed to identify the dental condition on the digital 3D model. Thus, the quality measure of the generated digital 3D model may contribute to the overall confidence score of the digital dental workflow.

The method may further comprise identifying, on the digital 3D model and based on the scan data, the dental condition, and determining the probability value that may represent the likelihood that the dental condition is identified correctly. The probability value may, alternatively or additionally, represent the likelihood that the dental condition belongs to a certain dental condition class.

Various types of algorithms for detecting dental conditions (referred to as dental condition algorithms) such as image evaluation algorithms or machine learning algorithms may be employed to evaluate the digital 3D model and/or the received scan data, and thereby to identify presence and/or severity of dental conditions on the digital 3D model. Identified dental conditions on the digital 3D model may be reflective of dental conditions in the actual dental situation.

The probability value may, in an example, be a part of an output of a trained neural network for identifying the dental condition. In another example, the probability value may be derived from similarity of contrast profiles of potential dental conditions and predetermined contrast profiles of dental conditions. A contrast profile of a dental condition may be generated by plotting pixel values of those pixels of the scan data representing part of the dentition suffering from the dental condition. The pixels of the scan data, for example pixels of the near-infrared images or enhanced diagnostic images, representing part of the dentition suffering from the dental condition may be manually selected by the user.

The contrast profile of the potential dental condition may be generated by plotting pixel values of those pixels of a near-infrared image or an enhanced diagnostic image representing part of the dentition with suspected dental condition. The pixels for plotting may be manually marked by the user on the corresponding near-infrared image or the enhanced diagnostic image, for example in form of a line, a circle or any other shape.

The predetermined contrast profile of the dental condition may be obtained by averaging a plurality of previously determined contrast profiles of the dental condition.

Further, the method may comprise determining the confidence score of the digital dental workflow based on the quality measure of the received scan data, the quality measure of the generated digital 3D model and/or the probability value.

The confidence score of the digital dental workflow may, in one example, be determined based only on the quality measure of the received scan data. In this case the confidence score reflects the quality of input data for the digital dental workflow. A high quality of input data may be a measure of a well-performed scanning process. The user may enable determination and visualization of the confidence score determined solely on the quality measure of the received scan data.

In another example, the confidence score may be determined based on the quality measure of the received scan data and on the quality measure of the generated digital 3D model, which may be mutually dependent. In this case the user may be provided with additional information about the digital dental workflow. For example, a low value for the quality measure of the received scan data may lower the quality measure of the generated digital 3D model. For example, the digital 3D model may be generated with partially missing color information, which may affect the quality measure of the generated digital 3D model and potentially influence identification of the dental condition.

The confidence score of the digital dental workflow may be determined, in an example, based on the quality measure of the received scan data and on the probability value representing the likelihood that the dental condition is identified correctly. In this case, a direct relationship between the received scan data and the output of the digital dental workflow may be established. A high value for the quality measure of the received scan data and a low probability value may convey a message to the user that the scanning process has been performed successfully but that the algorithm for identifying the dental condition may not be performing optimally. In this case an alternative algorithm for identifying the dental condition may be activated. An example of a digital dental workflow where the confidence score may be determined in this manner is when the dental condition is identified directly on the received scan data, for example when caries or tooth wear are identified in 2D images of the scan data, for example in near-infrared images and/or in color images.

In a further example the confidence score of the digital dental workflow may be determined based on the quality measure of the received scan data, the quality measure of the generated digital 3D model and on the probability value representing the likelihood that the dental condition is identified correctly. In this case, the user may be provided with complete feedback, from the input stage to the output stage of the digital dental workflow. Information such as quality of input data, quality of the generated 3D model and efficiency of the algorithm to detect the dental condition may be provided.

The confidence score of the digital dental workflow may reflect aggregated quality measures from different stages of the digital dental workflow such as receiving scan data, generating the digital 3D model and identifying the dental condition on the digital 3D model. The confidence score of the digital dental workflow may reflect the influence of different workflow stages on the end result. The user, for example a dentist, may be provided with information on how much the scan data may influence the generated digital 3D model and/or how much does the scan data influence identifying the dental condition. The user, having such information, may be able to improve certain stages of the workflow, for example by re-acquiring scan data or by modifying algorithm parameters for identifying the dental condition.

According to the disclosure, an intraoral scanner system configured to determine the confidence score of the digital dental workflow is presented, wherein the intraoral scanner system comprises:
- an intraoral scanner configured to obtain scan data representative of the dental situation,
- a display configured to display the digital 3D model of the dental situation,
- a processor configured to:
- receive scan data representative of the dental situation,
- determine the quality measure of the received scan data,
- generate the digital 3D model based on the received scan data,
- determine the quality measure of the generated digital 3D model,
- identify the dental condition on the digital 3D model based on the scan data, and determine the probability value that may represent the likelihood that the dental condition is identified correctly,
- determine the confidence score of the digital dental workflow based on the quality measure of the received scan data, the quality measure of the generated digital 3D model and/or the probability value,
- display the digital 3D model with the dental condition, and
- display the confidence score of the digital dental workflow.

A computer-implemented method for determining the confidence score of the digital dental workflow is further disclosed, the method comprising:
- receiving scan data representative of the dental situation,
- determining the quality measure of the received scan data,
- generating the digital 3D model based on the received scan data,
- determining the quality measure of the generated digital 3D model,
- identifying, on the digital 3D model and/or based on the scan data, the dental condition and determining a confidence score associated with the identified dental condition,
- determining the confidence score of the digital dental workflow based on at least one of the quality measure of the received scan data, the quality measure of the generated digital 3D model and the confidence score associated with the identified dental condition,
- displaying the digital 3D model with the dental condition, and
- displaying the confidence score of the digital dental workflow.

According to another embodiment of the disclosure, the intraoral scanner system configured to determine the confidence score of the digital dental workflow is presented, wherein the intraoral scanner system comprises:
- the intraoral scanner configured to obtain scan data representative of the dental situation,
- the display configured to display the digital 3D model of the dental situation,
- the processor configured to:
- receive scan data representative of the dental situation,
- determine the quality measure of the received scan data,
- generate the digital 3D model based on the received scan data,
- determine the quality measure of the generated digital 3D model,
- identify the dental condition on the digital 3D model based on the scan data, and determine the confidence score associated with the identified dental condition,
- determine the confidence score of the digital dental workflow based on the quality measure of the received scan data, the quality measure of the generated digital 3D model and/or the confidence score associated with the identified dental condition,
- display the digital 3D model with the dental condition, and
- display the confidence score of the digital dental workflow.

The confidence score associated with the identified dental condition may be a neural network confidence score, determined based on output of the trained neural network for identifying the dental condition.

The confidence score of the digital dental workflow may be displayed in a number of ways, including but not limited to, a numerical form, a text value, a color overlay, a pattern overlay and/or by using symbols. The numerical form of representing the confidence score of the digital dental workflow may be, for example, a number from 0 to 100 or a normalized value from 0 to 1. The text value for representing the confidence score of the digital dental workflow may be "low", "medium", or "high". The confidence score of the digital dental workflow may be aligned and displayed with the digital 3D model. The confidence score of the digital dental workflow may comprise a value per facet, point, surface and/or tooth of the digital 3D model and may thus be displayed on the corresponding region of the digital 3D model.

The confidence score, when displayed, provides an user (for example a dentist) with a general reliability metric of the digital dental workflow, considering some, or all individual stages of the workflow. The user may, based on the confidence score value, decide whether to trust the output of the digital dental workflow or whether a corrective action should be taken. In case of a low value of the confidence score, the user may decide to re-scan the patient, re-load the scan data and/or re-run the diagnostic process, for example with modified parameters. In one example, an alert may be generated and displayed if the confidence score is lower than a pre-defined threshold value for the confidence score. The pre-defined threshold may be manually set up.

In an example, if the confidence score is lower than the pre-defined threshold, any one or more of the intermediate quality measures and/or the probability value may be displayed. The quality measure of the received scan data, the quality measure of the enhanced diagnostic images, the quality measure of the generated digital 3D model and/or the probability value representing the likelihood that the dental condition is identified correctly may be displayed, indicating to the user which stage of the digital dental workflow contributes most to the confidence score. For example, the lowest value of the quality measure of the received scan data, the quality measure of the enhanced diagnostic images, the quality measure of the generated digital 3D model and/or the probability value may be displayed, if it is determined that the confidence score is below the threshold value for the confidence score. In an example, a recommendation to the user for a corrective action may be generated and displayed based on the lowest value of the quality measure of the received scan data, the quality measure of the enhanced diagnostic images, the quality measure of the generated digital 3D model and/or the probability value.

In an example, each of the intermediate quality measures and the probability value may have their specific threshold values. In case the quality measure of the received scan data, the quality measure of the enhanced diagnostic images, and/or the probability value are lower than their respective thresholds, an alert may be generated indicating the quality measure or the probability value below the respective threshold.

In an example, the confidence score may be logged for the purpose of performance tracking of the intraoral scanner system over time. Data log created in this way may be specifically useful for regulatory compliance where transparency and traceability are important.

In an example, data corresponding to low confidence score values may be flagged for manual review and annotation, which can be fed back into the trained neural network for improvement. This contributes to system performance improvement because the intraoral scanner system may in this case learn from uncertain cases.

In an embodiment of the disclosure, the intraoral scanner system configured to determine the confidence score of the digital dental workflow is presented, wherein the intraoral scanner system comprises:
- the intraoral scanner configured to obtain scan data representative of the dental situation,
- the display configured to display the digital 3D model of the dental situation,
- the processor configured to:
- receive scan data representative of the dental situation,
- determine the quality measure of the received scan data,
- generate the digital 3D model based on at least part of the received scan data,
- generate the enhanced diagnostic images based on at least the near-infrared images of the scan data,
- determine a quality measure of the enhanced diagnostic images,
- identify the dental condition in the dental situation based on the enhanced diagnostic images,
- determine the probability value representing the likelihood that the dental condition is identified correctly,
- determine the confidence score of the digital dental workflow based on the quality measure of the received scan data, the quality measure of the enhanced diagnostic images and/or the probability value,
- display the digital 3D model with the dental condition, and
- display the confidence score of the digital dental workflow.

In a further embodiment of the disclosure, the intraoral scanner system configured to determine the confidence score of the digital dental workflow is presented, wherein the intraoral scanner system comprises:
- the intraoral scanner configured to obtain scan data representative of the dental situation,
- the processor configured to:
- receive scan data representative of the dental situation, wherein the scan data comprises color images and near-infrared images,
- determine the quality measure of the received scan data,
- generate the enhanced diagnostic images based on at least the near-infrared images of the scan data,
- determine a quality measure of the enhanced diagnostic images,
- identify the dental condition in the dental situation based on the enhanced diagnostic images,
- determine the probability value representing the likelihood that the dental condition is identified correctly,
- determine the confidence score of the digital dental workflow based on the quality measure of the received scan data, the quality measure of the enhanced diagnostic images and/or the probability value.

The processor may further be configured to:
- generate the digital 3D model based the color images of the scan data,
- display the digital 3D model with the dental condition, and
- display the confidence score of the digital dental workflow.

Part of the scan data, for example the color images, may be used to generate the digital 3D model. The color images may comprise the required geometric data for constructing the digital 3D model.

Geometric data may comprise depth information relating to a camera position. Depth information may, in some cases, be inferred from recorded timestamps associated with images in the scan data. In a further example, depth information may be deduced by observing parts of the color images of the scan data that are in focus.

The geometric data may be continuously determined during the scanning process with the intraoral scanner. It may alternatively be obtained during dedicated periods of the scanning process, those periods being referred to as a "3D scan mode" where a white Light Emission Diode (LED) of the intraoral scanner may be operational.

Color images may be 2D images comprising color information of scanned teeth and/or gingiva and may be obtained by scanning the dental situation of the patient by means of the intraoral scanner equipped for recording color. This color information may be expressed through Red-Green-Blue (RGB) intensity values.

Fluorescence images may be 2D images comprising fluorescence information of scanned teeth and/or gingiva and may be obtained by scanning the dental situation of the patient by means of the intraoral scanner equipped for recording fluorescence. For example, the intraoral scanner may comprise an image sensor capable of detecting fluorescence emitted from fluorescent material when excited by a blue LED at a wavelength such as 405 nanometres.

Near-infrared (NIR) images may be 2D images obtained by scanning the dental situation of the patient by means of the intraoral scanner equipped with a NIR LED emitting light in a near-infrared spectrum, for example at 940 nm.

In an example, determining the quality measure of the received scan data may comprise determining a signal-to-noise ratio of color images.

Additionally or alternatively, determining the quality measure of the received scan data may comprise determining a signal-to-noise ratio of fluorescence images.

Additionally or alternatively, determining the quality measure of the received scan data may comprise determining a signal-to-noise ratio of near-infrared images.

Signal-to-noise ratio may be a good measure for determining the quality measure of the received scan data as it may reflect different lighting conditions present during obtaining of the scan data. Different thresholds for the signal-to-noise ratio may be defined and the images of the scan data may be categorized based on the signal-to-noise ratio value falling within the defined thresholds.

In an example, the quality measure of the received scan data may be one of "high", "medium", "low", based on the value of the signal-to-noise ratio. For example, value "high" may be assigned to the received scan data in case of signal-to-noise ratio higher than, or equal to 40:1. A value "medium" may be assigned to the received scan data in case of signal-to-noise ratio between 10:1 and 40:1. A value "low" may be assigned to the received scan data in case of signal-to-noise ratio being lower than 10:1. In general, the specific limits of signal-to-noise ratio values that separate the different categories may depend on the application and the overall expected quality of the scan data. If, for instance, all signal-to-noise ratio values are expected to be less than 10, then values above 8 may be considered "high" while values between 2 and 8 may be considered "medium".

In examples where the dental condition, for example caries, is identified based solely on the near-infrared images, it may be sufficient to only calculate the signal-to-noise ratio of the near-infrared images to obtain the quality measure of the received scan data. In this case the received scan data may only be the near-infrared images.

In examples where only fluorescence images are used to identify the dental condition, for example plaque, tooth cracks and/or caries, quality measure of the received scan data may be obtained by only calculating the signal-to-noise ratio of the fluorescence images.

In examples where the dental condition is identified by processing the digital 3D model by means of the trained neural network for identifying the dental condition, trained on variety of training data, then the quality measure of the received scan data may be determined by calculating the signal-to-noise ratio of the near-infrared images, fluorescence images, color images and/or geometric data.

The quality measure of the received scan data may comprise determining an amount of overlap between images of different modalities. Different modalities may refer to different scanning modalities, such as fluorescence scanning modality, color scanning modality and/or near-infrared scanning modality of the intraoral scanner.

Thus, an amount of overlap may be determined between the color images and the fluorescence images, or between the color images and the near-infrared images, or between the fluorescence images and the near-infrared images, or between the color images, the fluorescence images and the near-infrared images.

Overlap between images may refer to geometrical overlap. Determining the amount of overlap may thus comprise identifying a feature, such as an edge of a tooth or a cusp, in images of different modalities, and determining whether the images of different modalities with the identified feature overlap.

In one example, determining the quality measure of the received scan data may comprise determining the amount of overlap of the digital 3D model and the fluorescence images and/or color images overlaid onto the digital 3D model. In this way, a surface area of the digital 3D model with missing fluorescence overlay and/or color overlay may be quantified. A large surface area without the fluorescence overlay and/or color overlay may be result of the low quality measure of the received scan data. One or more threshold values for the surface area may be set up for determining the quality measure of the received scan data.

In one example, determining the quality measure of the received scan data may comprise determining a noise level in a sub-scan or in a series of color images that may be converted into the sub-scan. The sub-scan may represent a geometry of a part of the digital 3D model and may be generated from the series of color images of the scan data. In this was the quality measure of the received scan data may be determined per building block of the digital 3D model because a plurality of sub-scans is fused together to form the digital 3D model. If the quality measure of the received scan data, determined in this manner, does not satisfy a criterion, then the corresponding sub-scan may be discarded. A visual signal may be presented to the user to indicate that re-scanning of a part of dental situation may be needed.

A relationship between the noise level in the sub-scan and the quality measure of the received scan data may be defined, such as a liner function or a step function. Generally, the higher the noise level is, the lower the quality measure of the received scan data is.

One measure of the noise level in the sub-scan may be surface roughness. It may be assumed that tooth surfaces are not rough and that the tooth surfaces have a low average roughness (Ra) value. For example, the quality measure of the received scan data may have the value "low" if the average value of the surface roughness in the sub-scan is over 1.6 micrometres. Different threshold values may be set for the surface roughness average value to represent different quality measures of the received scan data.

A further measure of the noise level in the sub-scan may be related to identifying in-focus information in images of an object, where the imagers are obtained by scanning the object with a focus scanning intraoral scanner. This identification of in-focus information may be used to derive depth information for constructing a digital 3D model of the scanned object, given known parameters of the optical system of the focus scanning intraoral scanner. A reference signal (a pattern), such as a checkerboard pattern, may be projected onto the object being scanned and then recognized in the recorded images to determine which part of the recorded images is in-focus. A correlation measure may be defined between the reference signal projected by the intraoral scanner onto the object being scanned, and a recorded light signal by the intraoral scanner. An amplitude of the correlation measure may be used to determine the quality measure of the received scan data. The amplitude of the correlation measure may be directly related to a contrast in the projected checkerboard pattern. The checkerboard pattern may comprise dark and bright parts, referred to as "checkers". If the contrast between dark and bright checkers is high, the corresponding region of an image may be in focus. In an example, to evaluate the quality of the signal, an amplitude-to-texture value may be used, which relates the obtainable contrast to the general intensity of the reflected signal.

In a further example, determining the quality measure of the received scan data may comprise mutually comparing color images of the sub-scan. Comparing color images of the sub-scan may comprise comparing corresponding color intensity values of color images. Corresponding color intensity values may refer to color intensity values of corresponding pixels in the color images. Depending on the variation in color intensity values, the quality measure may be assigned. A large variation in color intensity values may result in a low quality measure of the received scan data. Thus, one or more threshold values for the variation in color intensity of the color images may be set up based on which the sub-scan quality may be assessed. Corresponding color intensity values may refer to color intensity values of those pixels representing the same dental object, for example the same tooth cusp, in the color images.

The variation in color intensity of color images corresponding to the sub-scan may be assessed depending on a scan angle at which the images were captured. Generally, in cases of the large variation in color intensity values, depending on the scan angle, the quality measure of the received scan data may be low, for example may be assigned value "low". An example of large variation in color intensity values depending on the scan angle may be observed with tooth cusps that appear as dark spots when seen from the scan angle such that there is no dentin layer behind the enamel layer of the tooth. These dark spots may falsely be identified as caries. When the scan angle is such that the dentin layer is behind the enamel layer, the cusps appear brighter. Thus, the color overlay on the generated digital 3D model may not be fully reliable in representing actual color of the dentition. This may be reflected in the quality measure of the received scan data and displayed to the user.

The method may further comprise determining the variation in the color intensity values of color images corresponding to the sub-scan. The color intensity values may, in an example, be weighted by the scan angle.

In an embodiment of the disclosure, determining the quality measure of the received scan data may comprise identifying an artifact, for example a glare effect in at least one of the near-infrared images or images derived from near-infrared images. Glare effects may be understood as undesirable objects of high brightness.

The glare effect may be identified by comparing a contrast profile of a potential glare effect to a predetermined contrast profile of the glare effect.

The quality measure of the received scan data may comprise determining an amount of glare effects in the near-infrared images or images derived from the near-infrared images. This may be a count of glare effects in all of the near-infrared images in the received scan data. Alternatively, the amount of glare effects in the near-infrared images or images derived from the near-infrared images may be determined per sub-scan. A threshold value for the amount of glare effects may be defined in determining the quality measure of the received scan data.

In some cases, measures to remove the identified glare effects may be undertaken. Therefore, determining the quality measure of the received scan data may comprise, in an example, detecting a removal of the glare effect in at least one of the near-infrared images or images derived from the near-infrared images, or detecting a total number of glare effect removals.

The removal of the glare effect may comprise combining near-infrared images of a region for which the removal of the glare effect is desired, wherein combining is performed for the near-infrared images of at least two sub-scans. The near-infrared images that are combined, and the corresponding sub-scans, may be partially overlapping. The combination of near-infrared images of different sub-scans may then result in the glare effect being reduced or completely removed.

Combining of the near-infrared images may be performed for those near-infrared images for which the region with the glare effect is in focus.

Determining the quality measure of the received scan data may be performed in real-time during acquisition of the scan data, during receiving of the scan data and/or during generation of the digital 3D model. This is advantageous as the user may be immediately presented with this quality measure, for example on the display screen or as an audio signal.

Alternatively, determining the quality measure of the received scan data may be performed in post-processing stage after generation of the digital 3D model. An advantage of real-time quality measure determination is that the quality measure of the received scan data may be visualized simultaneously with the generation of the digital 3D model, enabling the user to re-scan certain areas of the dental situation and thereby improve the quality measure, thereby also improving the overall digital dental workflow.

The method may further comprise displaying the determined quality measure of the received scan data.

The quality measure of the received scan data may be displayed in a numerical form, for example as a number from 0 to 100. Alternatively, the quality measure of the received scan data may be displayed in a text form such as "low", "medium", or "high".

The quality measure of the received scan data may, in an example, be displayed as a color overlay on the digital 3D model. Different colors may be used to represent different values for the quality measure. This may be of particular advantage to the user as the parts of the digital 3D model with low quality scan data can be directly highlighted. For example, the parts of the digital 3D model with missing color information or with high noise levels may be overlaid with red or orange color.

In some cases, it may be desirable to determine the quality measure of the received scan data representing a tooth. This may be the case if the dental condition is detected on the tooth level. Therefore, determining the quality measure of the received scan data may comprise identifying a part of the scan data representing the tooth and determining the quality measure of the part of the scan data representing the tooth.

Identifying the part of the scan data representing the tooth may comprise use of a trained neural network for segmenting the scan data. The trained neural network for segmenting the scan data may identify, in the color images of the scan data, pixels representing the tooth. The trained neural network for segmenting the scan data may be a convolutional neural network (CNN) adapted to perform a segmentation task on pixels of the scan data such as color images.

The trained neural network for segmenting the scan data may be trained on training data comprising annotated near-infrared images, annotated color images and/or annotated fluorescence images. This training data may be annotated by human experts such that different teeth and gingiva are annotated (marked).

The quality measure of the received scan data may be determined and/or displayed per tooth of the digital 3D model. In this way the user may observe specific teeth on the digital 3D model that may be highlighted due to insufficient quality measure of the received scan data.

In an example, determining the quality measure of the received scan data may be performed based on a series of images of the scan data acquired within a predetermined timeframe. The predetermined time frame may be set such that the acquired series of images correspond to a tooth. In an example, the predetermined timeframe may be in a range of 1 second to 5 seconds.

In another example, determining the quality measure of the received scan data may be performed based on a number of color images used to construct a sub-scan. A low number of color images used to construct the sub-scan may directly translate to a low quality measure of the received scan data.

The quality measure of the received scan data may be performed by a dedicated software algorithm referred to as image analyser algorithm.

It is mentioned that the scan data may comprise the near-infrared images obtained using the near-infrared light source. Based on at least the near-infrared images of the scan data, the enhanced diagnostic images may be obtained. For example, by combining images of different modalities composed images may be obtained. Or, by enhancing the contrast of near-infrared images, optimized near-infrared images may be obtained. The enhanced diagnostic images may thus be 2D images generated based on at least the near-infrared images of the scan data.

Composed images may be generated to enhance contrast of near-infrared images and thereby to improve detection of the dental condition, such as caries and more specifically proximal caries. The visibility of dental condition lesions is enhanced by generating the composed images, compared to observing the same dental condition lesions in the near-infrared images, color images and/or fluorescence images. Composed images may offer better contrast between the dental condition, such as caries lesions, and the tooth structure such as enamel or dentin.

In an example, composed images may be generated by combining intensity levels of pixels of the image sensor in the intraoral scanner, the pixels being associated with different wavelengths. For example, at a first time period, the image sensor unit may capture light information that relates to visible wavelength, for generating color images. At a second time period, the image sensor unit may capture light information that relates to a near-infrared wavelength, for generating near-infrared images. The intensity levels of the two time periods may be recorded and combined for enhancing the dental condition. The combination of the intensity levels may be done digitally by subtraction and/or addition of the intensity levels. In another example, the intensity levels may be captured and recorded during at least three time periods for at least three different wavelengths, such as white-coloured wavelength, blue-coloured wavelength and near-infrared wavelength.

The trained neural network for segmenting the scan data may be trained on training data comprising annotated images derived from near-infrared images, also referred to as annotated enhanced diagnostic images.

The enhanced diagnostic images may comprise optimized near-infrared images which may be generated by applying a contrast enhancement algorithm to the near-infrared images. This may be a non-linear enhancement algorithm such as a histogram equalization or an adaptive histogram equalization.

When the histogram of an image, such as near-infrared image or a color image, is equalized, all pixel values of the image are redistributed so that there are approximately equal number of pixels to each of the user-specified output grayscale classes (e.g., 32, 64, and 256). Contrast may be increased at the most populated range of brightness values of the histogram (or "peaks"). The contrast enhancement algorithm may automatically reduce the contrast in very light or dark parts of the image associated with the tails of a normally distributed histogram. Histogram equalization may separate pixels into distinct groups, if there are few output values over a wide range. Histogram equalization may be effective for original images having poor contrast to start with, such as near-infrared images in which caries, fillings, and/or cracks are to be identified.

In the adaptive histogram equalization, the image may be divided into sections, such as rectangular domains, wherein an equalizing histogram is determined and modified for each of the sections, so that lightness values of the pixels are redistributed in the image.

In another example, the contrast enhancement algorithm may be a linear enhancement algorithm like a piecewise contrast stretching that is applied to each piece of intensity intervals of the pixels. For example, pixels with intensities between a minimum and a maximum intensity are linearly scaled to for example 0 and 1 or 0 to 255 etc, without changing the pixels.

The contrast enhancement algorithm may be one or more of following linear contrast enhancement algorithms or a combination of two or more of the following linear contrast enhancement algorithms:
- Minimum-Maximum Linear Contrast Stretch (MMLC),
- Percentage Linear Contrast Stretch (PLC) and
- Piecewise Linear Contrast Stretch (PWLC).

The contrast enhancement algorithm may be one or more of following non-linear contrast enhancement algorithms or a combination of two or more of the following non-linear contrast enhancement algorithms:
- Histogram distribution (HD),
- Histogram Equalizations (HE),
- Adaptive Histogram Equalization (AHE) and
- Homomorphic Filters (HF) including a combination of low pass and high pass filtering.

The contrast enhancement algorithm may be a combination of one or more of the non-linear contrast enhancement algorithms and one or more of the linear contrast enhancement algorithms.

The contrast enhancement algorithm may include determining a histogram distribution of a plurality of pixels. The histogram distribution may include a distribution of a plurality of pixel values of the plurality of pixels, wherein the plurality of pixel values is within a first pixel value range. The algorithm may further determine a mean pixel value based on the plurality of pixel values which is subtracted from each of the plurality of pixel values, and then, truncating one or more of the plurality of pixels which has a pixel value that is outside three standard deviations off zero, wherein the three standard deviations include pixel values above the mean pixel value, or, both above and below the mean pixel value. The example where the three standard deviations include only positive pixel values or only pixel values above the mean pixel value may be defined as a half histogram distribution, and, the example where the three standard deviations include both positive and negative pixel values, or pixel values of both above and below the mean pixel value may be defined as a full histogram distribution. The contrast enhancement algorithm may then extend the pixel values of the truncated histogram distribution such that the pixel values of the truncated histogram distribution are within a second pixel value range, and wherein the second pixel value range is larger than the first pixel value range. The second pixel value range may be between 0 and 255, 0 and 511 etc.

The three standard deviations is an example, however, the three standard deviations could be replaced by a single standard deviation, two, four, five standard deviations.

The truncation and the extended distribution of the remaining pixels of the plurality of pixels over the second pixel value range results in an improved contrast of the optimized near-infrared images.

The method according to an embodiment may comprise determining the quality measure of the enhanced diagnostic images. This quality measure may be determined in any of the manners previously stated for determining the quality measure of the received scan data.

Acquisition of the scan data of different modalities may occur during periods of different duration. For example acquisition of near-infrared images may occur during a period that is different from a period for acquisition of color images or fluorescence images. Therefore, color images, fluorescence images and near-infrared images may have their corresponding acquisition cycle.

Determining the quality measure of the received scan data may be performed for a series of color images acquired during an acquisition cycle for the color images which may be in a range of 150 to 250 milliseconds, for example 200 milliseconds.

Determining the quality measure of the received scan data may additionally or alternatively be performed for a series of fluorescence images acquired during an acquisition cycle for the fluorescence images.

Determining the quality measure of the received scan data may additionally or alternatively be performed for a series of near-infrared images acquired during an acquisition cycle for the near-infrared images.

Determining the quality measure of the generated digital 3D model may comprise determining a surface area of the generated digital 3D model for which color information is missing. As mentioned before, intraoral scanning may be performed in a sub-optimal manner such that received scan data is incomplete. In some cases, color information or fluorescence information may be missing on parts of the digital 3D model.

The determined surface area with missing color information may be compared to a first surface area threshold. Depending on the comparison result, the quality measure of the generated digital 3D model may be assigned.

Alternatively, a ratio of the determined surface area of the generated digital 3D model for which color information is missing to the complete surface area of the generated digital 3D model may be determined. Based on this value, the quality measure of the generated digital 3D model may be assigned.

Similar process may be performed with respect to fluorescence information. Determining the quality measure of the generated digital 3D model may comprise determining a surface area of the generated digital 3D model for which fluorescence information is missing.

The determined surface area of the generated digital 3D model for which fluorescence information is missing may be compared to a second surface area threshold. Depending on the comparison result, the quality measure of the generated digital 3D model may be assigned.

A ratio of the determined surface area of the generated digital 3D model for which fluorescence information is missing to the complete surface area of the generated digital 3D model may be determined. Based on this value, the quality measure of the generated digital 3D model may be assigned.

The quality measure of the generated digital 3D model may be displayed on the digital 3D model. This quality measure may be displayed as a color overlay, a pattern overlay, as a numerical value or a plurality of numerical values overlayed over the digital 3D model, as a text message and/or as an audio signal.

The dental condition in the dental situation of the patient may be identified based on the scan data, the optimized diagnostic images and/or the generated 3D model. Identifying the dental condition my comprise applying a trained neural network for identifying the dental condition to the scan data, the optimized diagnostic images and/or to the generated digital 3D model, or applying a non-neural network algorithm to the scan data, the optimized diagnostic images and/or to the generated digital 3D model.

The probability value representing the likelihood that the dental condition is identified correctly may be provided as an output of the trained neural network for identifying the dental condition. For example, it may be a value between 0-1 given as a result of the SoftMax activation function used in the final layer of the trained neural network for identifying the dental condition.

Identifying the dental condition using the non-neural network algorithm may comprise identifying an interproximal caries lesion by comparing a contrast profile of a potential interproximal caries lesion to a predetermined contrast profile of the interproximal caries lesion. In this case, the probability value may be determined based on a fitting error obtained after fitting the contrast profile of the potential interproximal caries lesion to the predetermined contrast profile of the interproximal caries lesion. The same approach may be applied to dental indications other than interproximal caries.

The contrast profile of the potential interproximal caries lesion may be generated by plotting pixel values of those pixels of the near-infrared images or enhanced diagnostic images representing part of the dentition that potentially suffers from caries. The pixels of the near-infrared images, representing part of the dentition potentially suffering from the dental condition may be manually selected by the user. Instead of the near-infrared images, enhanced diagnostic images may be used.

The predetermined contrast profile of the interproximal caries lesion may be obtained by averaging a plurality of previously determined contrast profiles of the interproximal caries lesions.

In another example, a trained neural network for classifying contrast profiles into contrast profiles of known dental indications (such as interproximal caries lesions, caries lesions or tooth cracks) may be used. This neural network may be trained on different predetermined contrast profiles of dental indications. The trained neural network may classify input contrast profile into one of the output classes based on criteria such as profile shape, profile length, profile depth, color and other. Thus, when applied to an input contrast profile of the potential dental indication, the trained neural network may classify the input contrast profile into a contrast profile of interproximal caries, a contrast profile of caries or a contrast profile of a dental crack. The trained neural network for classifying contrast profiles may be a convolutional neural network.

Identifying the dental condition may comprise identifying dental plaque based on red (R) and green (G) channel values of fluorescence information in the fluorescence images. Various functions of red and green channel values may be employed to estimate plaque presence. Exemplary functions may be a difference R-G or ratio (R-G)/(R+G).

The probability value may be displayed, for example in a numerical form or as a color overlay over the digital 3D model.

Identifying the dental condition may comprise identifying a type of the dental condition, a position of the dental condition on the digital 3D model, a severity of the dental condition, a volume of the dental condition and/or a surface area of the dental condition.

At the end of the digital dental workflow the confidence score of the digital dental workflow may be determined. This confidence score may be determined based on the quality measure of the received scan data, the quality measure of the enhanced diagnostic images, the quality measure of the generated digital 3D model and/or the probability value representing the likelihood that the dental condition is identified correctly. The confidence score may thus give an insight into the overall efficiency of the digital dental workflow, as well as insight into how much every stage of the digital dental workflow affects the end result.

In some examples, the confidence score of the digital dental workflow may be determined based on any one or more of the quality measure of the received scan data, the quality measure of the enhanced diagnostic images, the quality measure of the generated digital 3D model and the probability value representing the likelihood that the dental condition is identified correctly.

The determined quality measure of the received scan data, the quality measure of the enhanced diagnostic images, the quality measure of the generated digital 3D model and the probability value may all affect the confidence score differently. Thus, a weighting system may be introduced in order to modify the effect of these individual parameters of the confidence score of the digital dental workflow.

A first weight coefficient may be applied to the quality measure of the received scan data.

A second weight coefficient may be applied to the quality measure of the generated digital 3D model.

A third weight coefficient may be applied to the probability value representing the likelihood that the dental condition is identified correctly.

A fourth weight coefficient may be applied to the quality measure of the enhanced diagnostic images.

The first weight coefficient, the second weight coefficient, the third weight coefficient and/or the fourth weight coefficient may be derived empirically. In an example, it may be desired to emphasize influence of a low quality measure of the received scan data, the quality measure of the generated digital 3D model, the probability value and/or the fourth weight coefficient on the confidence score. This may be achieved by assigning a high value for the first weight coefficient, the second weight coefficient, the third weight coefficient and/or the fourth weight coefficient, respectively.

In one example, the user may value the received scan data as the most critical element in the digital dental workflow. Namely, low quality of the scan data may lead to inaccurate conclusions on those scan data. In such case, the first weight coefficient may be set up to have a higher value than the second weight coefficient, the third weight coefficient and/or the fourth weight coefficient.

The confidence score of the digital dental workflow may be displayed, for example in numerical form, as a color and/or pattern overlay over the digital 3D model.

In one embodiment of the disclosure a threshold for the probability value representing the likelihood that the dental condition is identified correctly may be adjusted by the user. Adjusting the threshold for the probability value may affect, for example, when a certain label is assigned to a facet of the digital 3D model. Setting a low threshold for the probability value may result in tagging a large number of facets with the dental condition. Setting a high threshold for the probability value may result in tagging fewer facets with the dental condition. If applied to the trained neural network for identifying the dental condition, the trained neural network may be made more "aggressive" in case of the low threshold value is set, or more "conservative" in case of setting the high threshold value.

In an embodiment of the disclosure, a computer-implemented method for determining a neural network confidence score is disclosed, the method comprising:
- receiving the digital 3D model representative of the dental situation,
- identifying the dental condition on at least a part of the digital 3D model using the trained neural network for identifying the dental condition, wherein the trained neural network for identifying the dental condition has been trained on training data comprising a plurality of annotated training samples,
- determining the neural network confidence score associated with the identified dental condition, and
- displaying the neural network confidence score.

An annotated training sample may be a digital 3D model of a dental situation or a part of a digital 3D model of a dental situation that has been annotated manually for presence of a dental condition. The training data may comprise over 300 annotated training samples, preferably over 1000 annotated training samples.

In an embodiment, the training data may be synthetic data comprising a plurality of artificially generated digital 3D models. The artificially generated digital 3D models may comprise artefacts such as brackets and/or retainers. The artificially generated digital 3D models may, additionally or alternatively, comprise tooth conditions such as recession, tooth wear, plaque, malocclusion, gingivitis, or other. The artificially generated digital 3D models may be generated using generative network models such as Variational Autoencoders (VAEs), Generative Adversarial Networks (GANs), and/or normalizing flows. Such artificially generated dataset can be extremely useful for training purposes where training samples or variability in training samples is lacking. Such dataset may be quick to produce, may be customizable based on requirements of the problem, and may address the problem of lack of data. Further, such dataset may be cost effective as just few annotations to augment the dataset are needed to generate much more data using the dataset. Moreover, artificially generated training data is not confronted with data privacy issues as the data is artificial, even though it looks like real data. Furthermore, the problem of data imbalance is resolved, as variability may be introduced into the training data. Finally, more generalizable data may be created, thus resolving the issue of generalizability by having few samples from different ethnic groups, religions, genders etc.

Input to a generative network model may comprise digital 3D models without artefacts and/or tooth conditions and digital 3D dental models with the intended artefacts and/or tooth conditions. Representation of the intended artefacts and/or tooth conditions may be learned by the generative network model. In case realistic digital 3D models are given as the input, a partially-synthetic dataset may be created. Completely synthetic data can be created by providing, as the input to the generative network model, digital 3D models with the intended artefact and/or tooth condition.

Further, the intraoral scanner system configured to determine the neural network confidence score is disclosed, wherein the intraoral scanner system comprises:
- the intraoral scanner configured to obtain scan data representative of the dental situation,
- the display configured to display the digital 3D model of the dental situation,
- the processor configured to:
- receive the scan data,
- generate the digital 3D model based on the received scan data,
- identify the dental condition on the at least part of the digital 3D model using the trained neural network for identifying the dental condition, wherein the trained neural network for identifying the dental condition has been trained on training data comprising the plurality of annotated training samples,
- determine the neural network confidence score associated with the identified dental condition, and
- displaying the neural network confidence score.

It may be pointed out that the neural network confidence score is not an equivalent, nor a synonym to, the probability value representing the likelihood that the dental condition is identified correctly.

In an example, the neural network confidence score may be determined based on analysing the training data used for training of the trained neural network for identifying the dental condition. In this way under-represented cases, referred to also as "special" cases may be identified in the training data. By identifying these cases, the "weak points" of the trained neural network for identifying the dental condition may be identified. The neural network confidence score may thus indicate that an input case to the trained neural network for identifying the dental condition may be of a special case type and that the trained neural network for identifying the dental condition has not been sufficiently trained on that type of input.

Analysing the training data may comprise determining a distribution of the training data.

The distribution of the training data may be a distribution of the training samples according to a number of teeth per training sample. In this way the digital 3D models that are under-represented in the training data may be identified. For example, the training data may have few training samples with 5 teeth or less. This may be a hint that, if the trained neural network for identifying the dental condition is applied to a case (a 3D model) with 5 teeth, the output of the trained neural network for identifying the dental condition may have a low associated neural network confidence score.

The distribution of the training data may be a distribution of the training samples according to, for example, presence of retainers, braces, preparations, fillings, restorations and/or third molars. By determining the distribution of the training data an outlier in the training data may be identified.

The outlier may be training sample comprising retainers, brackets, a preparation for a restoration, a filling, a restoration, a third molar, and/or a number of teeth less than a threshold.

In one example, a latent space representation of the outlier may be generated. Generating the latent space representation of the outlier may comprise encoding the outlier into a neural network such as an autoencoder network.

A latent space representation may be generated for every outlier that has been identified. In this way, a plurality of clusters in the latent space may be obtained, where each cluster of the plurality of clusters represents a type of under-represented training sample in the training data. The generated latent space representations may thereby represent a "list" of special cases that may affect the neural network confidence score.

The latent space representation of the digital 3D model that is input into the trained neural network for identifying the dental condition may be generated. Furthermore, the latent space representation of the digital 3D model may be compared to the latent space representation of the outlier to obtain a similarity measure. The similarity measure may be a linear distance of the latent space representation of the digital 3D model to the latent space representation of the outlier, or a linear distance of the latent space representation of the digital 3D model to the latent space representation of the cluster representing the outlier.

The neural network confidence score may be assigned based on the obtained similarity measure.

The method according to an embodiment may further comprise:
- applying the trained neural network for identifying the dental condition to a test data comprising a plurality of test samples,
- identifying a test sample from the plurality of test samples satisfying a criterion,
- determining the neural network confidence score based on the correspondence of the test sample and the digital 3D model that is input into the trained neural network for identifying the dental condition.

The criterion may comprise determining that a percentage of the area of the test sample correctly classified is below a threshold. The test sample may be stored in a confidence score list.

A performance plot may be generated during application of the trained neural network for identifying the dental condition to the test data and a latent space cluster for part of the test data resulting in poor performance may be generated. The performance may be judged, for example, by determining a percentage of correctly classified area test samples and comparing the percentage to a set tolerance. For example, good performance may be assigned if the percentage of a correctly classified area is over 98% for 90% of test samples in the test data.

Test data may be used to evaluate the performance of the trained neural network for identifying the dental condition. The test data may comprise test samples. A test sample may be a digital 3D model of a dental situation or a part of a digital 3D model of a dental situation.

In an embodiment, a quality measure for test performance of the trained neural network for identifying the dental condition may be determined. The test performance of the trained neural network may relate to performance of the trained neural network based on the test data. The quality measure for test performance may then be used to determine the neural network confidence score. For example, if the quality measure for test performance is low, then the neural network confidence score may be assigned low value.

The latent space representation of the digital 3D model that is input into the trained neural for identifying the dental condition network may be generated.

The latent space representation of the digital 3D model may be compared to the latent space cluster to obtain the similarity measure.

The neural network confidence score may be based on the obtained similarity measure.

A computer program product is disclosed comprising instructions which, when the program is executed by a computer, causes the computer to carry out any one or more methods according to the disclosure.

A computer readable medium is further disclosed comprising instructions which, when executed by a computer, cause the computer to carry out any one or more methods according to the disclosure.

A data processing apparatus is further disclosed comprising means for carrying out any method according to the disclosure.

An intraoral scanning system is further disclosed comprising means for carrying out any one or more methods according to the disclosure.

### Brief description of the figures

FIGS. 1A and 1B schematically illustrate steps of exemplary digital dental workflows for identifying a dental condition;
FIGS. 2A - 2E illustrate examples of determining the quality measure of received scan data;
FIGS. 3A and 3B illustrate how the quality measure of the received scan data and/or the generated digital 3D model may be visualized;
FIG. 4 illustrates a 3D scan mode and a diagnostic scan mode operation of the intraoral scanner, defining time periods for which the quality measure of the received scan data may be determined;
FIGS. 5A and 5B illustrate examples of determining a probability value representing a likelihood that the dental condition is identified correctly;
FIG. 6 illustrates an example of determining the confidence score of the digital dental workflow;
FIG. 7 illustrates an example of the trained neural network for identifying a dental condition;
FIG. 8 illustrates a further example of the trained neural network for identifying the dental condition;
FIG. 9 illustrates an example of determining a neural network confidence score;
FIG. 10 illustrates an intraoral scanner system according to the disclosure.

### Detailed description

In the following description, reference is made to the accompanying figures, which show by way of illustration how the invention may be practiced.

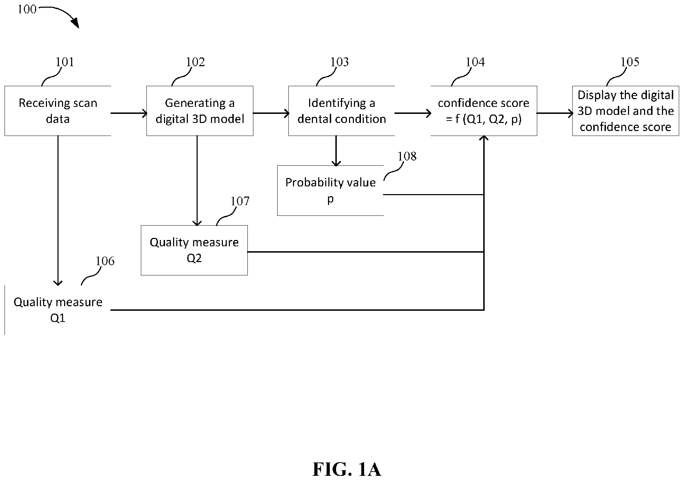

FIG. 1A illustrates a flowchart of a digital dental workflow (100) in which a dental condition is identified. In step 101 a scan data representative of a dental situation of a patient may be received, for example by a processor of an intraoral scanner system 1000.

The scan data may comprise geometric data, color images, fluorescence images and/or near-infrared images. Color images, fluorescence images and near-infrared images may be referred to as images of different scan modalities. For example, an intraoral scanner 1001 may be able to acquire color images, fluorescence images and/or near-infrared images of the dental situation by engaging different scan modalities comprising light sources emitting light at different wavelengths.

Geometric data may be comprised in the color images of the scan data and may be used for constructing the digital 3D model representative of the dental situation. Geometric data may comprise depth information relating to a camera position.

The geometric data may be continuously acquired during the scanning with the intraoral scanner 1001. It may alternatively be obtained during dedicated parts of the scanning process, those parts being referred to as a "3D scan mode" where a white Light Emitting Diode (LED) of the intraoral scanner, or a LED within the visible light spectrum may be operational.

Color images may be 2D images comprising color information of scanned teeth and/or gingiva and may be obtained by scanning the dental situation of the patient by means of the intraoral scanner 1001 equipped for recording color. This color information may be expressed through Red-Green-Blue (RGB) intensity values.

Fluorescence images may be 2D images comprising fluorescence information of scanned teeth and/or gingiva and may be obtained by scanning the dental situation of the patient by means of the intraoral scanner 1001 equipped for recording fluorescence. For example, the intraoral scanner 1001 may comprise an image sensor capable of detecting fluorescence emitted from fluorescent material when excited by a blue or ultraviolet light, for example at a wavelength of around 400 nanometres, such as 405 nanometres. The fluorescent material may be bacteria metabolites present in the dental situation. Fluorescence information present in the fluorescence images may indicate presence of dental conditions such as plaque, caries, tooth cracks, tartar or other.

Near-infrared (NIR) images may be 2D images obtained by scanning the dental situation of the patient by means of the intraoral scanner 1001 equipped with a NIR LED, emitting light with a wavelength in range of 780 nm to 2500 nm, for example at 940 nm. Near-infrared images may be used to detect dental conditions such as tooth cracks, caries within the volume of teeth and/or interproximal caries occurring in the regions between the teeth.

According to the disclosure a quality measure 106 (Q1) of the received scan data may be determined. This quality measure 106 (Q1) is a parameter that may be considered in determining the confidence score 607 of the digital dental workflow 100. The quality measure 601 (Q1) may also be displayed to the user upon being determined, thereby giving the user valuable information on quality and useability of the received scan data.

Step 102 illustrates generating the digital 3D model 300 based on a part of the received scan data, for example based on the color images of the scan data. The digital 3D model 300 may usually be generated even if the scan data is incomplete. For example, some fluorescence information and/or color information may be missing from a specific part of the dental situation due to sub-optimal scanning techniques. The digital 3D model 300 may nevertheless be generated but its accuracy and the accuracy of any prediction of the dental indications on such digital 3D model 300 may be questionable.

Determining a quality measure 107 (Q2) of the generated digital 3D model 300 may further be performed. The quality measure 107 (Q2) of the generated digital 3D 300 model may relate, for example, to completeness of the geometric information, color images and/or fluorescence images available for generating the digital 3D model 300.

The quality measure 107 (Q2) may be visualized, advantageously presenting the user with a visual overview of how suitable the digital 3D model 300 may be for detecting the dental condition. For example, missing geometric information may result in empty spots (holes) in the digital 3D model 300 which should not be evaluated for the dental condition presence. This kind of missing information can be detected and presented to the user.

The quality measure 107(Q2) may be a further parameter in determining the confidence score 607 of the digital dental workflow 100, in addition to the quality measure 106 (Q1).

**In** step 103 the dental condition may be identified based on the scan data and/or the digital 3D model 300. **In** some examples, a trained neural network 700, 800 for identifying the dental condition may be applied either to the scan data directly, or to the digital 3D model 300 to identify the dental condition. The dental condition may be caries, plaque, tartar, tooth crack, dental recession, tooth wear, oral cancer and/or gingivitis.

Furthermore, a probability value 108 (p) that may represent a likelihood that the dental condition is identified correctly may be determined. In some cases this probability value 108 (p) may be a part of the output of the trained neural network 700, 800 for identifying the dental condition, such as a value of a SoftMax activation function. In other cases, the probability value may be computed by comparing a parameter, for example a contrast profile, of the identified dental condition to a reference parameter.

In step 104 the confidence score 607 of the digital dental workflow 100 may be determined. The confidence score 607 of the digital dental workflow 100 may be based on the quality measure of the received scan data 106 (Q1), the quality measure of the generated digital 3D model 107 (Q2), the quality measure of enhanced diagnostic images and/or the probability value 108 (p).

The confidence score 607 of the digital dental workflow 100 may be displayed in a numerical form, for example as a number from 0 to 100 or as a normalized value in a range 0 to 1. Alternatively, the confidence score of the digital dental workflow may be displayed as a text saying "low", "medium", or "high". The confidence score 607 may, additionally or alternatively, be displayed as a color or pattern overlay on the digital 3D model (300).

This confidence score 607 of the digital dental workflow 100 may reflect the probability with which the dental condition is identified, considering the quality measure 106 (Q1) of the received scan data, the quality measure 107 (Q2) of the generated digital 3D model and/or the probability value 108 (p). A comprehensive insight into the performance of the digital dental workflow 100 may be obtained if all parameters Q1, Q2 and p are considered in the confidence score determination. If fewer parameters are considered in the confidence score determination, still a sufficient insight may be obtained in the performance of a particular one or more intermediate step of the digital dental workflow.

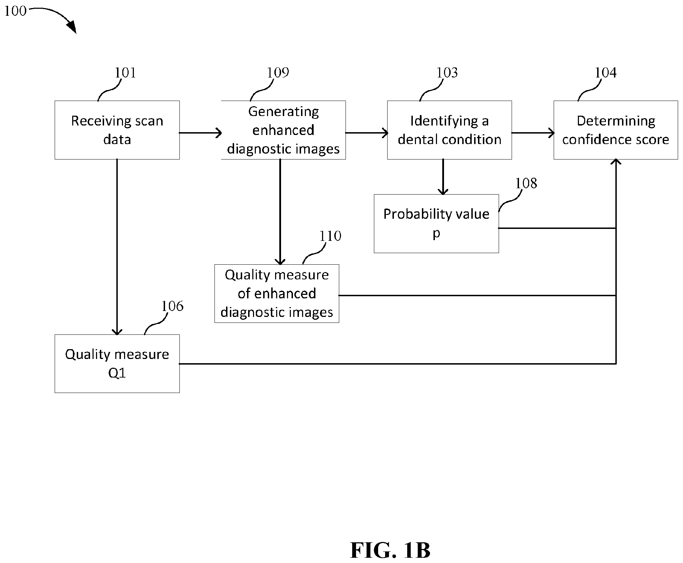

FIG. 1B illustrates a flowchart of another exemplary digital dental workflow (100) in which the dental condition is identified. In step 101 the scan data representative of a dental situation of the patient may be received, for example by a processor of an intraoral scanner system 1000. The scan data may comprise the color images and the near-infrared images.

The quality measure 106 (Q1) may be determined for the received scan data, as previously presented for FIG. 1A.

Further, enhanced diagnostic images may be generated based on the at least near-infrared images of the scan data (illustrated in step 109). The enhanced diagnostic images may comprise composed images and/or optimized near-infrared images generated by previously presented methods. Once generated, these images offer improved contrast of dental conditions and allow for more efficient identification of dental conditions.

A quality measure of the enhanced diagnostic images may be performed for the enhanced diagnostic images (step 110). In an example, the quality measure of the enhanced diagnostic images may comprise determining an amount of glare effects (unwanted spots of high brightness) in the enhanced diagnostic images per sub-scan. Alternatively or additionally, the quality measure of the enhanced diagnostic images may comprise determining an overlap between the color images and the enhanced diagnostic images. Furthermore, the quality measure of the enhanced diagnostic images may be determined in any manner as the quality measure of the received scan data.

The dental condition may be identified based on the enhanced diagnostic images (illustrated in step 103 of FIG. 1B). In an example, identifying the dental condition in the dental situation based on the enhanced diagnostic images may comprise comparing a contrast profile of a potential dental condition to a predetermined contrast profile of the dental condition. For example, the potential dental condition may be marked on the enhanced diagnostic image by a line (or any other form) which additionally extends over neighbouring surface of the potential dental condition. The pixel values corresponding to the line (or any other form) may be plotted, highlighting the profile of the potential dental condition. This profile may be compared to the predetermined contrast profile of the dental condition, which may be derived by averaging historical contrast profiles of the dental condition and may be stored in the memory of the intraoral scanner system 1000.

In step 108 of FIG. 1B the probability value p, which may represent a likelihood that the dental condition is identified correctly, may be determined. This value p may be determined based on a fitting error obtained after fitting the contrast profile of the potential dental condition to the predetermined contrast profile of the dental condition. Generally, the better the fit between the two profiles is, the higher the value p is.

Step 104 of FIG. 1B illustrates determining the confidence score of the digital dental workflow 100 based on the quality measure of the received scan data, the quality measure of the enhanced diagnostic images and the probability value p. The confidence score may be a numerical value obtained by various functions of the numerical values for the quality measure of the received scan data, the quality measure of the enhanced diagnostic images and the probability value p, such as a product, an average, a weighted average etc. In another example, the confidence score may be determined as one of the text values "high", "medium", "low" by selecting the lowest of the text values for the quality measure of the received scan data, the quality measure of the enhanced diagnostic images and the probability value p.

The confidence score may be displayed to the user, for example correlated to the digital 3D model (300) of the patient's dental situation.

A significant advantage may be gained by determining quality measures at various stages of the digital dental workflow 100. By displaying one or more of the determined quality measures, the user may be timely informed about potential issues in the digital dental workflow 100 stages, allowing time for intervention and improvement. As a result, the efficiency of the whole workflow may be improved and the time which the patient spends in the dentist office may be reduced.

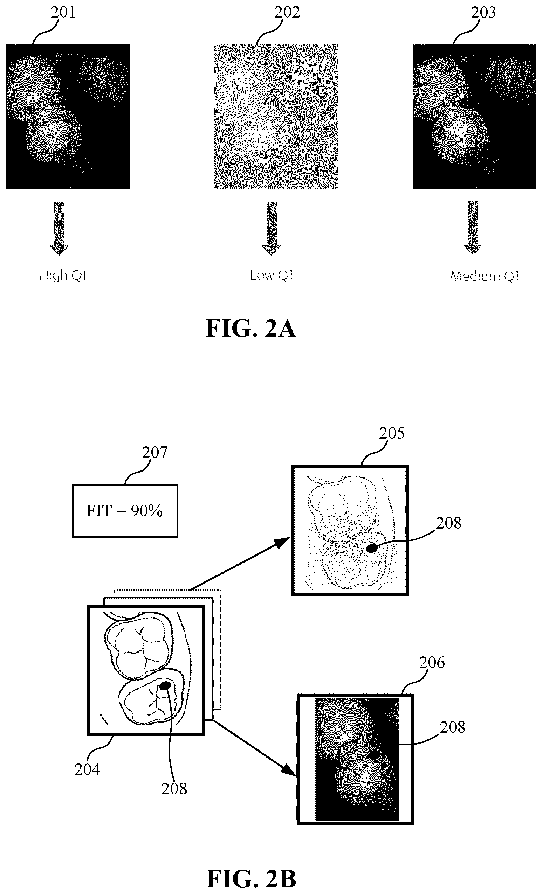

FIG. 2A-2E illustrate examples of determining the quality measure 106 (Q1) of received scan data.

Determining the quality measure 106 (Q1) of the received scan data may comprise determining a signal-to-noise ratio of color images, fluorescence images and/or near-infrared images.

Examples of the near-infrared images 201, 202, 203 with different signal-to-noise ratio values are shown in FIG. 2A. Image 201 has the highest signal-to-noise ratio, thereby may be assigned a value "high" as the quality measure (Q1) value. Image 202 has a low signal-to-noise ratio and may therefore be assigned value "low" as the quality measure (Q1) value. Image 203 has a signal-to-noise ratio in between that of images 201 and 202 and may be assigned value "medium" as the quality measure (Q1) value.

In an example, value "high" for the quality measure (Q1) may be assigned to an image of the received scan data in case of signal-to-noise ratio higher than, or equal to 40:1. A value "medium" may be assigned to the image of the received scan data in case of signal-to-noise ratio between 10:1 and 40:1. A value "low" may be assigned to the image of the received scan data in case of signal-to-noise ratio being lower than 10:1. In general, the specific limits of signal-to-noise ratio values that separate the different categories may depend on the application and the overall expected quality of the scan data. If, for instance, all signal-to-noise ratio values are expected to be less than 10, then values above 8 may be considered "high" while values between 2 and 8 may be considered "medium". The quality measure (Q1) of the overall received scan data may be determined as average of the quality measures of individual images forming the scan data.

Determining the quality measure 106 (Q1) of the received scan data may comprise determining an overlap between images of different modalities. This is illustrated in FIG. 2B where an overlap of images of three different modalities is illustrated, namely overlap of a color image 204, a fluorescence image 205 and a near-infrared image 206.

Overlap may refer to a geometric overlap of identified characteristic features in the images. A feature may be identified in one of the images 204, 205, 206, for example an edge of a tooth or a cusp of a tooth. Then, the same feature may be identified in other two of the images 204, 205, 206. The images may then be brought into a mutually overlapping stage and it may be determined whether the identified features are overlapping. Based on the amount of overlapping, the quality measure 106 (Q1) may be assigned. FIG. 2B indicates that an overlap measure 207 in this case is 90%, based on overlapping a characteristic feature 208 of images 204, 205, 206. The quality measure 106 (Q1) of the overall received scan data may be determined as average of the quality measures of individual images forming the scan data.

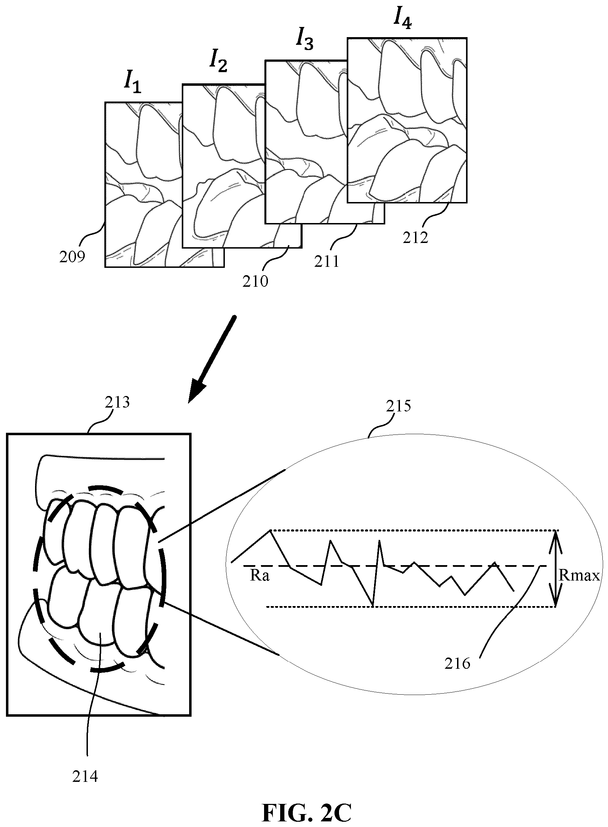

Determining the quality measure 106 (Q1) of the received scan data may comprise determining a noise level in a sub-scan 213, as illustrated in FIG. 2C. The sub-scan 213 may represent a geometry of a part of the digital 3D model. The sub-scan 213 may be generated from a series of color images 209, 210, 211, 212, of the scan data, illustrated for simplicity as four images I1-I4 on FIG. 2C. By merging a plurality of sub-scans 213 together, the complete digital 3D model 300 may be obtained.

One measure of the noise level in the sub-scan 213 may be a surface roughness 215. It may generally be assumed that tooth surfaces 214 are not rough and that the tooth surfaces 214 have a low average roughness 216 (Ra) value. For example, the quality measure of the received scan data may have the value "low" if the average value of the surface roughness 215 in the sub-scan 213 is over 1.6 micrometres. Different threshold values may be set for the surface roughness 215 average value to represent different quality measures of the received scan data.

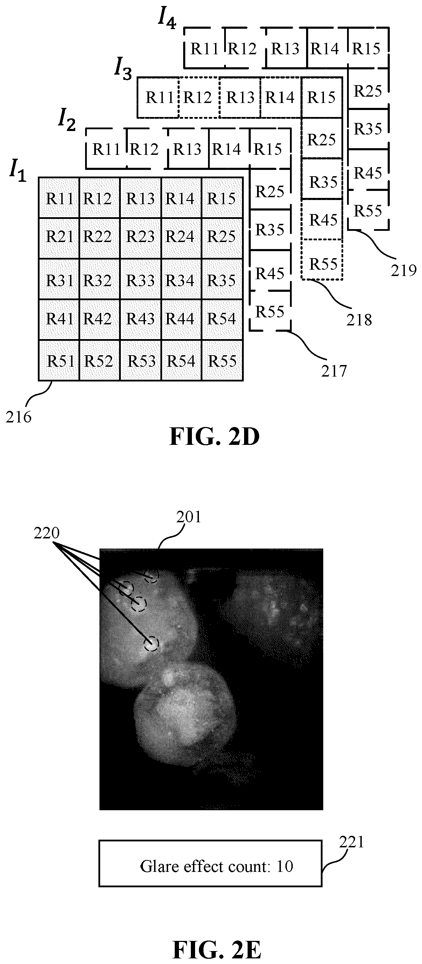

In a further example, illustrated in FIG. 2D, determining the quality measure 106 (Q1) of the received scan data may comprise mutually comparing color images 209, 210, 211, 212 of the sub-scan 213. Comparing color images 209, 210, 211, 212 of the sub-scan 213 may comprise comparing corresponding color intensity values of color images 209, 210, 211, 212. Comparison may be performed per Red Green Blue (RGB) color channel and such that corresponding pixel values of images 209, 210, 211, 212 are compared. FIG. 2D illustrates comparison of four images I1-I4 of the sub-scan 213 where corresponding pixel values in red channel are compared, for example R11 values of I1, I2, I3, I4 may be mutually compared. Depending on the variation in color intensity values, the quality measure 106 (Q1) may be assigned. A large variation in color intensity values may result in a low quality measure.

The variation in color intensity of color images 209, 210, 211, 212 corresponding to the sub-scan 213 may be assessed depending on a scan angle at which the images were captured. Generally, in cases of the large variation in color intensity values, depending on the scan angle, the quality measure of the received scan data may be low, for example may be assigned value "low". An example of large variation in color intensity values depending on the scan angle may be observed with tooth cusps that appear as dark spots when seen from the scan angle from which no dentin layer behind the enamel layer can be seen. These dark spots may falsely be identified as caries.

When the scan angle is such that the dentin layer is behind the enamel layer, the cusps appear brighter. Thus, the variation in color intensity values may be due to the scan angle and not due to caries lesions. For this reason, the final color on the generated digital 3D model 300 may be trusted less in identifying the dental condition such as the caries lesion.

The color intensity values may, in an example, be weighted by the corresponding scan angle in order for the effect of the scan angle on the color intensity values to be reduced.

Determining the quality measure 106 (Q1) of the received scan data may comprise identifying an artifact in at least one of the near-infrared images 201. As illustrated in FIG. 2E the artifact may be a glare effect 220. In general, the near-infrared images may comprise unwanted objects, captured by the image sensor unit of the intraoral scanner 1001, referred to as artifacts. These artifacts, such as glare effects, may contribute to false-positive identification of dental conditions such as caries, cracks or false-positive identification of dental fillings.

The glare effect 220 may be identified by comparing a contrast profile of a potential glare effect to a predetermined contrast profile of the glare effect. For example, the potential glare effect may be marked on the near-infrared image 201 or on the enhanced diagnostic image by a line (or any other form) which additionally extends over neighbouring surface to the potential glare effect. The pixel values corresponding to the line (or any other form) may be plotted, highlighting the profile of the potential glare effect. This profile may be compared to the predetermined contrast profile of the glare effect, which may be derived empirically and stored in the memory of the intraoral scanner system.

The quality measure 106 (Q1) of the received scan data may comprise determining an amount 221 of glare effects 220 in the near-infrared image 201. A count of glare effects 201 may be determined in all of the near-infrared images in the received scan data, in a single near-infrared image 201 in the received scan data, and/or in the series of near-infrared images corresponding to the sub-scan 213. A threshold value for the amount of glare effects 220 may be defined in determining the quality measure 106 (Q1) of the received scan data. The quality measure 106 (Q1) determined in this manner may directly define the usability of the near-infrared images for diagnostic purposes.

In some cases, measures to remove the identified glare effects 220 may be undertaken. Therefore, determining the quality measure 106 (Q1) of the received scan data may also comprise detecting a removal of the glare effect 220 in at least one of the near-infrared images 201 and/or detecting a total number of glare effect removals.

The user may manually trigger removal of the identified glare effects 220 which may result in the improved quality measure 106 (Q1) of the received scan data. In another example, the removal of the identified glare effects 220 may be triggered automatically, based on the determined quality measure 106 (Q1).

The removal of the glare effect 220 may comprise combining near-infrared images of different sub-scans, for example combining the near-infrared images of two sub-scans. The combination of the near-infrared images of different sub-scans may result in the glare effect 220 being reduced or completely removed because the region of one image with the glare effect 220 may be "merged" with the same region of another image without the glare effect 220. The region in this case may be a surface of a tooth in the near-infrared images. A required condition for combining of the near-infrared images is that the images to-be-combined cover the same region (surface).

In an example, the removal of the glare effect 220 may comprise combining the near-infrared image of the region comprising the glare effect 220 and the near-infrared image of the same region without the glare effect 220. To increase the likelihood that the combining is performed with the near-infrared image of the same region without the glare effect 220, images of different sub-scans may be used.

In a further example, the region in the near-infrared images that are combined may be in-focus to ensure better quality of the combined image.

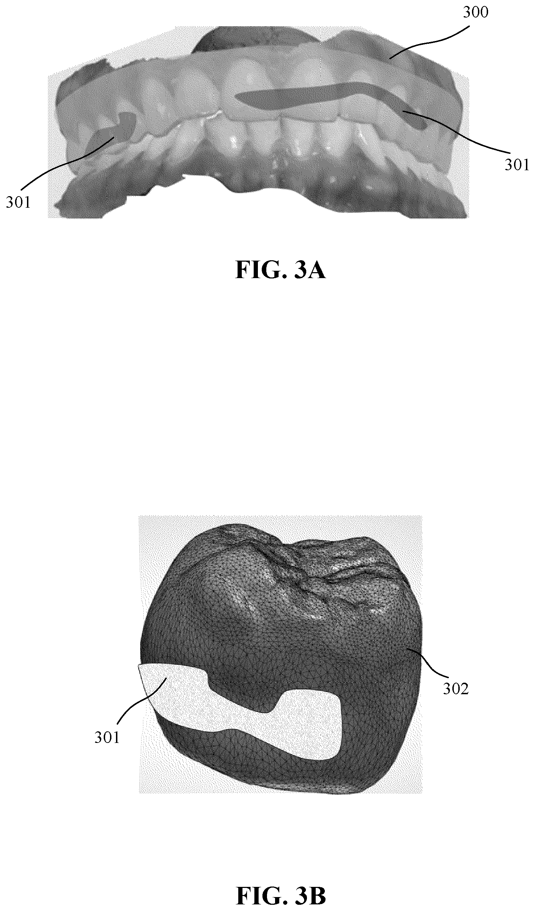

FIGS. 3A and 3B illustrate how the quality measure 106 (Q1) of the received scan data and/or the quality measure 107 (Q2) of the generated digital 3D model 300 can be visualized.

In FIG 3A the digital 3D model 300 is illustrated with a color overlay 301 that may indicate associated quality measure 106 (Q1) of the received scan data and/or a quality measure 107 (Q2) of the generated digital 3D model 300.

The color overlay 301 may be automatically placed over some of the tooth and/or surfaces of the digital 3D model 300. In an example, those tooth surfaces over which the color overlay 301 is placed may miss corresponding color information. In another example, those tooth surfaces may miss corresponding fluorescence information. In yet another example, the variation in the color intensity values and/or the surface roughness in those tooth surfaces may be such that the quality measure 106 (Q1) of the received scan data and/or the quality measure 107 (Q2) of the generated digital 3D model 300 are not sufficiently high, and the user should be able to visually notice it. Timely display of such overlay 301 may be effective as the user may then proceed to re-scan parts of the dental situation and improve the quality measure (Q1) of the received scan data and/or a quality measure (Q2) of the generated digital 3D model 300. Alternatively, the overlay 301 may be such as to indicate that the quality measure 106 (Q1) of the received scan data and/or the quality measure 107 (Q2) of the generated digital 3D model 300 is sufficiently good for the digital dental workflow to proceed to the next stage. By observing this message, the user may be confident that the scanning process was performed successfully.

The color overlay 301 may be placed on a single tooth 302 of the digital dental model 300, as illustrated in FIG. 3B. In this case, only certain facets of the tooth 302 may be colored to indicate that the quality measure 106 (Q1) of the received scan data and/or the quality measure 107 (Q2) for the tooth 302 is of certain value. The color overlay 301 on the tooth 302 may be advantageous as more specific information can be shown, related to facets of the digital 3D model 300 that may usually be difficult to observe on the whole model 300.

The intraoral scanner 1001 of the intraoral scanner system 1000 may be configured to acquire the color images, fluorescence images and/or near infrared images of the scan data continuously and simultaneously during the scanning process. Alternatively, the scanning process may comprise a dedicated period in which the color images may be acquired, a period in which the fluorescence images may be acquired, and a period in which the near-infrared images may be acquired. These periods may be different in duration and may overlap, partially overlap or may not overlap with each other.

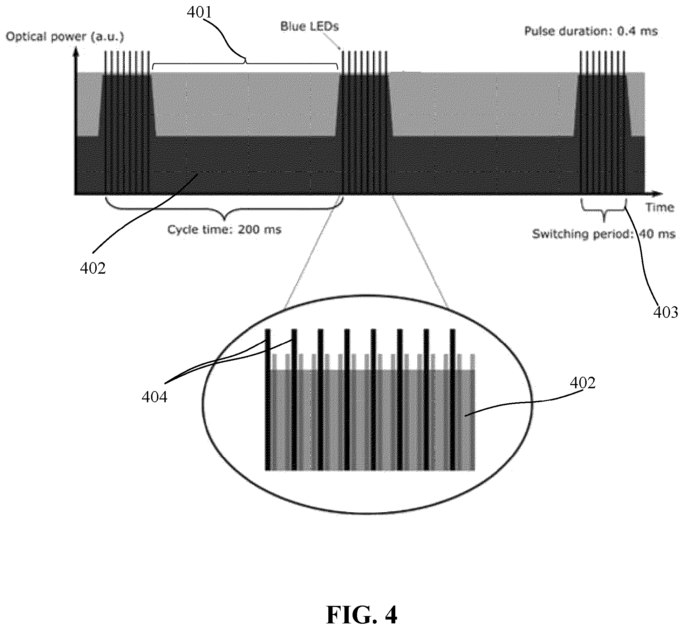

FIG. 4 illustrates a 3D scan mode 401 and a diagnostic scan mode 403 operation of the intraoral scanner 1001, defining time periods for which the quality measure 106 (Q1) of the received scan data may be determined. An example of operation of a white LED, a blue LED and a NIR LED in a scanning cycle is illustrated. The periods indicated in FIG. 4 may define how the quality measure 106 (Q1) of the received scan data and/or the quality measure 107 (Q2) of the generated digital 3D model 300 may be calculated.

**In** the scanning cycle illustrated, the white LED may be continuously operational during a time period indicated with a numeral 401, and may be responsible for generating color images and/or geometric information for generating the digital 3D model 300. This mode of the intraoral scanner may be referred to as "3D scan mode". During the time period 403, the processor may switch between the operation of the white LED and the operation of the blue LED.

The quality measure 106 (Q1) of the received scan data and/or the quality measure 107 (Q2) of the generated digital 3D model 300 may be determined for the 3D scan mode, i.e. for a part of the scanning cycle in which the white LED is continuously operational (period indicated with numeral 401).

The quality measure of the enhanced diagnostic images may be determined for the diagnostic scan mode, i.e. for a part of the scanning cycle in which the NIR LED is ON at the full power. In FIG. 4, the NIR LED is ON at all times, indicated with the numeral 402.

The blue LED, responsible for acquiring the fluorescence images may be operational during a shorter period of time 403. Pulses of the blue LED operation during the period 403 are indicated with numerals 404. During this same period of time 403, the NIR LED may also operate with the full power. This mode of intraoral scanner 1001 may be referred to as "diagnostic scan mode". The quality measure 106 (Q1) of the received scan data, with respect to the diagnostic scan mode in which the blue LED and NIR LED are operating with full power, may be determined for a period shorter than the scanning cycle, for example indicated as 40 milliseconds in FIG. 4 while the scanning cycle is 200 milliseconds. The NIR LED may operate with a power level less than full power, outside of the diagnostic scan mode. Alternatively, the NIR LED may be shut down when it is not used, i.e. outside of the diagnostic scan mode while the white LED may be operational at all times at a power level less than full power. Considerations on how to construct the scan cycle may involve factors like scanner power consumption, battery life, desired accuracy (level of detail) of the digital 3D model 300 and go beyond the current disclosure.

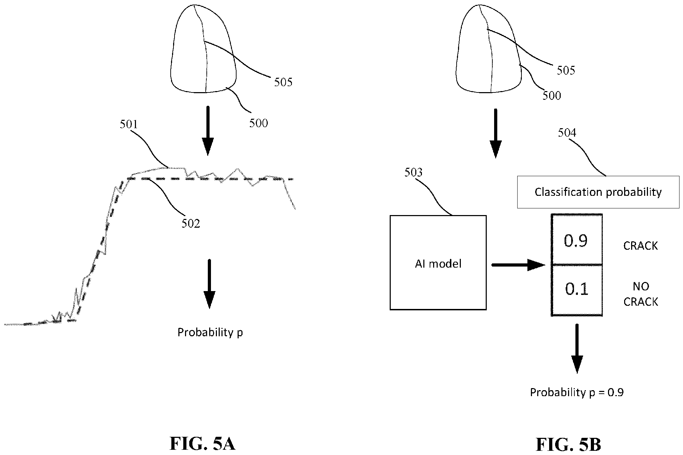

FIGS. 5A and 5B illustrate examples of a probability value representing a likelihood that the dental condition is identified correctly.

The dental condition may be identified in a number of different ways. For example, the scan data may be processed, and the dental condition may be identified on the scan data directly or on the enhanced diagnostic images that may be generated based on at least the near-infrared images of the scan data. Various algorithms such as artificial intelligence (AI) models or fitting algorithms may be used to identify the dental condition. The dental condition may also be identified on the digital 3D model 300 generated based on at least part of the received scan data. Again, an artificial intelligence (AI) model such as a trained neural network 700, 800 for identifying the dental condition may be used to process the digital 3D model 300, or a numerical algorithm may be used to identify the dental condition on the digital 3D model 300.

As illustrated in FIG. 5A, the probability value (p) representing the likelihood that the dental condition is identified correctly on a tooth 500 may be determined by comparing a contrast profile 501 of the dental condition to the predetermined contrast profile 502 of that dental condition. The dental condition in this case of FIG. 5 is the dental crack 505.

Depending on the similarity (fit) between the contrast profile 501 and the predetermined contrast profile 502, the probability value (p) may be assigned. The contrast profile 501 may be determined as described previously for the glare effect 220 of FIG. 2E.

In the example of FIG. 5B, the probability value (p) representing the likelihood that the dental condition is identified correctly on the tooth 500 may be given as an output 504 of an AI model 503, for example the trained neural network 700, 800 for identifying the dental condition. In this case a classification probability 504 value may be used as the probability value (p) in the determination of the confidence score 607 of the digital dental workflow 100. In FIG. 5B, classification probability 504 value has a value of 0.9 that the input (tooth 500) comprises the tooth crack 505. This value of 0.9 (90%) may be taken as the probability value (p).

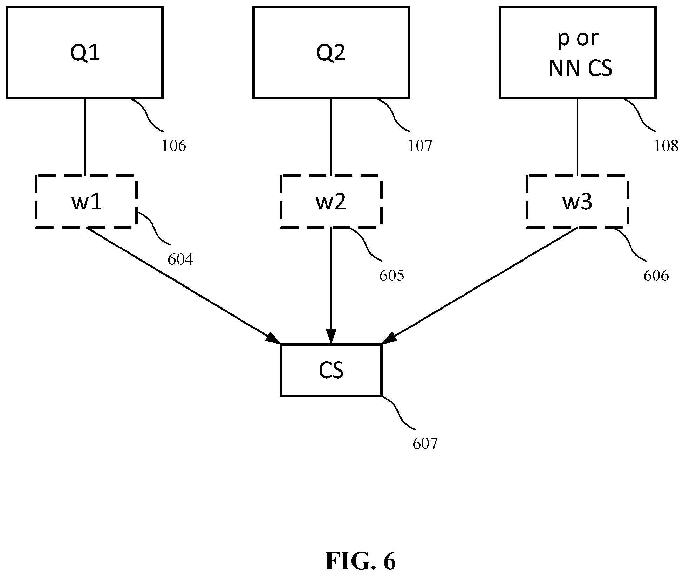

FIG. 6 illustrates an example of determining the confidence score 607 of the digital dental workflow 100.

The quality measure 106 (Q1) of the received scan data may be determined according to any previously presented method. This quality measure (Q1) may be a sole parameter in determining the confidence score 607 or may be one of the parameters used.

A first weight 604 (w1) may optionally be applied to the quality measure 106 (Q1) to increase or decrease the effect of this parameter on the overall confidence score 607 of the digital dental workflow 100.

The quality measure 107 (Q2) of the generated digital 3D model 300 may be determined according to any previously presented method. This quality measure 107 (Q2) may be a sole parameter in determining the confidence score 607 or may be used as one of the parameters in determining the confidence score 607 of the digital dental workflow 100.

A second weight 605 (w2) may optionally be applied to the quality measure 107 (Q2) to increase or decrease the effect of this parameter on the overall confidence score 607 of the digital dental workflow 100.

The probability value 108 (p) representing the likelihood that the dental condition is identified correctly may be determined according to the previously presented methods. This probability value 108 (p) may be a sole parameter in determining the confidence score 607 of the digital dental workflow 100 or may be one of the parameters used.

A third weight 606 (w3) may optionally be applied to the probability value 108 (p) to increase or decrease the effect of this parameter on the overall confidence score 607 of the digital dental workflow 100.

Instead of the probability value p a neural network confidence score may be utilized in the determination of the overall confidence score 607 of the digital dental workflow 100. This neural network confidence score may be a function of the probability value p in one example. In another example, the neural network confidence score may be derived by analysing training data used to train the trained neural network for identifying the dental condition, as will be explained later.

The quality measures 106, 107, the probability value 108 and the confidence score 607 of the digital dental workflow 100 may, in some examples, be presented in a numerical form or may be categorized as one of "high", "medium", "low", or similar.

In Table 1, an example of values for the quality measures Q1 106, Q2 107, the probability value p 108 and the resulting confidence score 607 are presented.

**Table 1.**

| Quality measure Q1 | Quality measure Q2 | Probability value p | Confidence score |
|---|---|---|---|
| low | medium | 0.5 (low) | low |
| medium | medium | 0.8 (medium) | medium |
| high | medium | 0.98 (high) | high |

In the example shown in Table 1, values for the various quality measures and the resulting confidence score are expressed as "low", "high", "medium". The probability value p may be expressed numerically and then classified into one of the "low", "high" or "medium" values based on the predefined ranges. For example, value "medium" for the probability value p may be assigned for p values in the range 0.7-0.85. The confidence score may be selected by taking majority of Q1, Q2, p as shown in the Table 1. Two "low" values in the first example will result in "low" confidence score value. Alternatively, the confidence score of the digital dental workflow may be determined by selecting a lowest value of the quality measure of the received scan data, the quality measure of the generated digital 3D model and the probability value p.

Alternatively, all of the quality measure values, the probability value p and the confidence score may be expressed numerically.

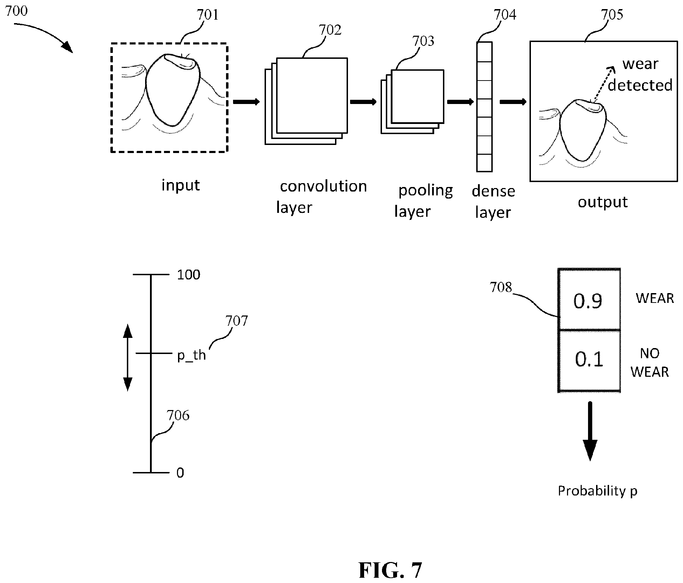

FIG. 7 illustrates an example of the trained neural network 700 for identifying the dental condition, in this case tooth wear.

The trained neural network 700 may be a convolutional neural network and may comprise a plurality of convolution layers 702 capable of capturing low-level features of an input 701, i.e. obtaining convolved features. Moreover, one or more of pooling layers 703 may be present, responsible for reducing the spatial size of convolved features. Convolutional neural networks may be particularly suitable for processing matrix information such as 2D images.

The input 701 may be in a 2D or 3D format and may comprise at least one input element such as a tooth, a facet of a tooth, a point of a tooth point cloud and/or a voxel. The input 701 may be a 2D image of a tooth of the jaw which has one of its surfaces affected by the dental condition, in this exemplary case tooth wear. The input 701 may alternatively comprise one or more of 2D images of a surface the tooth or at least one pixel of the 2D image of the tooth.

The output 705 may comprise the probability value 708 representing the likelihood that the dental condition is present in the input 701 and/or may classify the dental condition further into a severity category. For example, tooth wear may be detected in the input 701 and a moderate severity value may be assigned to it. Severity classes may also be "mild" or "severe", for example.

Behaviour of the trained neural network 700 may be modified by adjusting a threshold value (p_th) 707 for the probability value based on which detection of the dental condition may be established. By increasing the threshold value 707 (closer to maximum value 100) the trained neural network 700 may act more "conservative" and identify fewer dental condition lesions. By decreasing the threshold value 707 (towards the minimum value 0) the trained neural network 700 may act more "aggressive" and identify more dental condition lesions.

In some examples, adjusting of the threshold value 707 for the probability value (p) may be detected and the confidence score 607 of the digital dental workflow 100 may be adjusted based on adjusted threshold value 707.

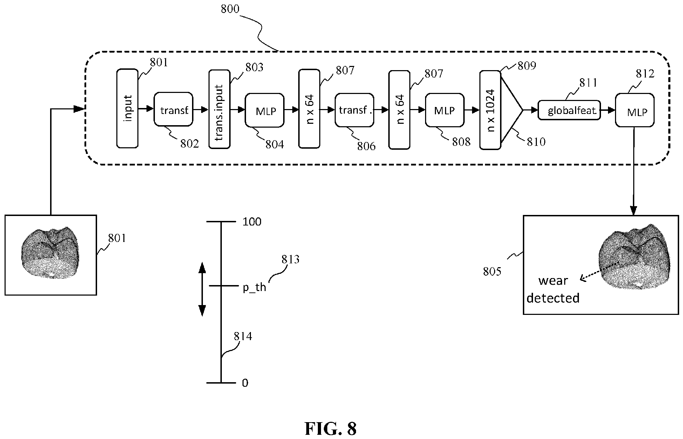

FIG. 8 illustrates a further example of the trained neural network 800 for detecting dental conditions.

The trained neural network 800 may be a PointNet neural network capable of processing at least part of the digital 3D model in form of a point cloud. Model presented in FIG. 8 is capable of taking an unordered point set as input. A generic model of this network architecture was developed by Stanford University ("PointNet: Deep Learning on Point Sets for 3D Classification and Segmentation", Charles R. Qi, Hao Su, Kaichun Mo, Leonidas J. Guibas). Additional to PointNet neural network, the trained neural network 800 may be a PointNet++ or a PointNet-like neural network.

A point cloud representation of a tooth of the digital 3D model 300 may serve as the input 801 to the trained neural network 800. The input 801 may be of a format n x 3, where n is a number of 3D points representing the tooth and dimension 3 stands for three coordinates of each point representing the tooth. The input 801 may be processed by an input transformation block 802 which may be a 3 x 3 rotation transformation. The input transformation block 802 has the effect of achieving invariance under transformation. Block 803 may be a transformed input with the format n x 3. Alternatively, the input 801 may be of a format n x 6, where n is the number of 3D points representing the tooth and dimension 6 stands for three coordinates of each point representing the tooth and three coordinates representing a normal of each point.

Block 804 may represent a first multi-layer perceptron with shared weights, which may process independently all 3D points for feature extraction. Its function is to achieve invariance under permutation of 3D points order. Architecture of the first multi-layer perceptron 804 (MLP) may comprise an input layer of 3 dimensions, a hidden layer of 64 neurons while an output layer may comprise 64 features. Block 807 may have dimension of n x 64.

Feature transformation block 806 may comprise a transformation matrix of dimensions 64 x 64, which may be a regularization matrix, for example of L2 type. Block 807 may be dimensioned as n x 64. Block 808 represents a second multi-layer perceptron with shared weights. Architecture of the second multi-layer perceptron 808 (MLP) may comprise an input layer of 64 features, two hidden layers of 64 neurons and 128 neurons, while an output layer of the second multi-layer perceptron 808 may comprise 1024 features. Block 809 may have dimension of n x 1024. In max pooling block 810, feature vectors of 3D points are aggregated to create a 1024-dimensional global feature representation 811 of the input 801. Block 812 represents a third multi-layer perceptron. Architecture of the third multi-layer perceptron 812 (MLP) may comprise an input layer of 1024 features, two hidden layers of 512 neurons and 256 neurons, while an output layer of the third multi-layer perceptron 812, also referred to as the output 805 of the trained neural network 800, may comprise classification scores.

The output 805 of the trained neural network 800 may comprise an indication that the dental condition, in this case tooth wear, is present in the input 801 and/or may determine a location of the tooth wear in the input 801. The output 805 may further classify the tooth wear into a tooth wear type, for example one of the abrasion, attrition or erosion.

In another example the output 805 may comprise additional information on the dental condition, including the severity level. In the case of tooth wear, additional information may include the number (ID) of the affected tooth, the number (count) of affected tooth surfaces, the tooth surface where the tooth wear lesion is located (e.g. occlusal surface) etc.

The threshold value (p_th) for the probability value, based on which the dental condition may be detected in case of the trained neural network 800 of FIG. 8, may be adjusted just as explained for FIG. 7.

Generally, the trained neural network 700, 800 for identifying the dental condition may be trained to detect the lesion of the dental condition directly on a digital 3D model 300 of teeth, or on 2D images of the digital 3D model of teeth.

The trained neural network 700, 800 for identifying the dental condition may, in an example, be a trained convolutional neural network (CNN) configured for detecting the dental condition such as caries, tooth wear, plaque, gingivitis and/or dental recession. The trained neural network 700, 800 for identifying the dental condition may be adapted to detect presence of the dental condition on a digital 3D model, but also to detect the severity of the dental condition on the digital 3D model.

The trained neural network 700, 800 for identifying the dental condition may be trained on training data comprising annotated training samples on which the dental condition presence and/or severity may be marked by a trained professional.

The neural network for identifying the dental condition may be trained for a classification task. A training process for the classification task of the trained neural network 700, 800 may be described as follows. The overall aim of a non-trained neural network may be to develop the trained neural network 700, 800 capable of classifying the input according to presence of the lesion of the dental condition and/or the severity value of the lesion.

The training process may be referred to as "supervised training" due to use of a training data that has been labelled (annotated). The training data may comprise at least 1000 training samples, for example three-dimensional digital models which can yield over 17000 individual teeth. The training data may be in 2D format, i.e. it may comprise a plurality of 2D training images of teeth or parts of teeth. The training data may alternatively be in 3D format comprising a plurality of teeth represented as 3D meshes, 3D graphs or point clouds.

The training data may comprise labels, also referred to as target data or ground truth data. The training data may thus comprise labels indicating presence and/or severity of the dental condition on the samples of training data. The annotating, also referred to as labelling, may be performed for example by a qualified dental practitioner. The training data may be labelled according to the dental condition presence, for example with "Yes" or "No" labels indicating the presence or absence of the dental condition. Additionally or alternatively, the training data may be labelled with the severity value labels associated with corresponding lesions.

The labels may be regarded as the target or the ground truth for a given training data set.

The training data may be fed into the non-trained neural network. Output of the non-trained neural network may be obtained, comprising a probability value indicating which output class the input, or an input element, belongs to.

A loss may be determined by comparing the output to the generated target data. This loss may be determined by a loss function. One example of the loss function may be a mean-squared-error function although various other loss functions may be suitable. In order to train the non-trained neural network, this loss function should be minimized. The minimization of the loss function may be achieved for example by using a stochastic gradient descent algorithm.

Through this process of minimizing the loss function, weights of the non-trained neural network may be updated.

The training process may be an iterative process in which the same training data set may be fed, iteratively, into the non-trained neural network until a stopping criterion is reached. The stopping criterion may be achieving a specific threshold value for the loss function. The stopping criterion may be a number of epochs (training cycles) after which performance metrics cease to increase.

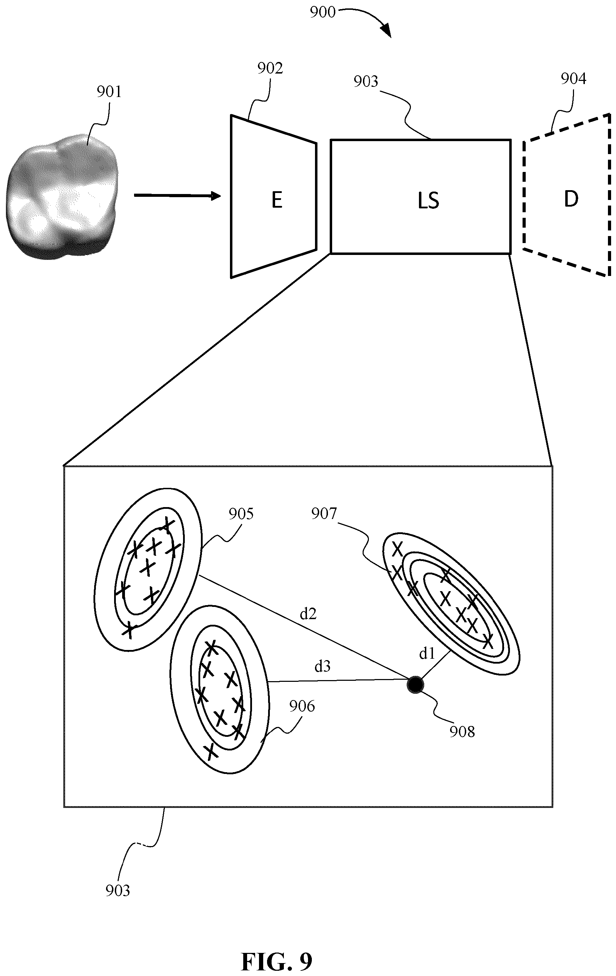

FIG. 9 illustrates an example of determining the neural network confidence score.

The neural network confidence score may be determined based on analysing the training data used to train the neural network 700, 800 for identifying the dental condition. Through a statistical analysis of the training data, under-represented cases, referred to also as "special cases", may be identified in the training data. By identifying these cases, the "weak points" of the trained neural network 700, 800 for identifying the dental condition may be identified. This means that this trained neural network may not perform reliably on similar cases because of insufficient training on such cases. This behaviour may be reflected by the neural network confidence score.

Analysing the training data may comprise determining a distribution of the training data.

The distribution of the training data may be performed, in an example, with respect to a number of teeth per training sample. In this way the samples in the training data that are under-represented with respect to the number of teeth may be identified. For example, the training data may have few samples with 5 teeth or less. This may mean that, if the trained neural network 700, 800 for identifying the dental condition is applied to a case with 5 teeth, the output of the trained neural network 700, 800 should have a low associated neural network confidence score because the trained neural network 700, 800 has not been trained on sufficient cases with 5 teeth.

The distribution of the training data may be performed, for example, with respect to presence of retainers, braces, preparations, fillings, restorations and/or third molars in the training data. By determining the distribution of the training data an outlier (under-represented case) in the training data may be identified.

The outlier may be a training sample comprising retainers, brackets, a preparation for a restoration, a filling, a restoration, a third molar, and/or a number of teeth less than a threshold.

A latent space representation of the outlier may be generated.

Illustrated in FIG. 9 is an encoder-decoder neural network 900 comprising an encoder 902 and optionally a decoder 904. The encoder-decoder neural network 900 may comprise a latent space 903. Generally, a latent space of a neural network may be understood as an embedding space comprising representations of input data in form of latent space variables, wherein those latent space variables resembling each other more closely are positioned closer to one another in the latent space relative to more differing latent space variables. The latent space variables may be scalar numbers.

The latent space 903 may comprise previously encoded samples (digital 3D models) that may be referred to as test data, based on which the performance of the trained neural network for detecting the dental condition may be tested. These encoded samples, more precisely their latent space variables indicated with "x" in FIG. 9, may be clustered in the latent space 903 in a plurality of clusters 905, 906, 907. Some of the clusters may thus represent special cases on which the trained neural network performs poorly - for example clusters 905 and 906. Clusters 905 and 906 may thus be referred to as low-confidence clusters and may represent, as an example, cases with braces and third molars, respectively.

The cluster 907 may represent a cluster of cases on which the trained neural network performs satisfactorily. The cluster 907 may thus be referred to as high-confidence cluster.

Once a digital 3D model 901, or at least a part of it, designated for input to the trained neural network for detecting the dental condition is encoded into the encoder-decoder neural network 900, its latent space representation 908 may be obtained.

The latent space representation 908 of the digital 3D model 901 may be compared to the low confidence cluster 905, 906 and/or to the high confidence cluster 907. Based on this comparison a similarity measure may be obtained based on which the neural network confidence score may be assigned.

The similarity measure may be a linear distance of the latent space representation of the digital 3D model 908 to the low confidence cluster 905, 906 and/or to the high confidence cluster 907.

Observing FIG. 9, the similarity measure may be based on the distance values d1, d2, d3 from the latent space representation 908 of the digital 3D model 901 to each of the clusters 905, 906, 907. Based on the distance value the neural network confidence score may be assigned. For example, in FIG. 9 the latent space representation 908 is closest to the high-confidence cluster 907 because the corresponding distance d1 is lower than distances d2 or d3 to the low-confidence clusters 905 and 906, respectively. In this case, the neural network confidence score may be set to "high".

A latent space representation may be generated for every outlier that has been identified in the training data or during testing of the trained neural network performance. In this way, a plurality of clusters in the latent space may be obtained, where each cluster of the plurality of clusters represents a type of under-represented samples in the training data. The generated latent space representations may thereby represent a mapping of special cases that may affect the neural network confidence score.

Performance of the trained neural network may be quantified by using the test data. One measure for quantifying the performance may be determining a percentage of the area in samples of the test data that is correctly classified. If the correctly classified area is below a threshold, the corresponding sample may be stored in a confidence score list.

A performance plot may be generated during applying of the trained neural network 700, 800 for identifying the dental condition to the test data. Subsequently, a latent space cluster for a part of the test data resulting in poor performance may be generated.

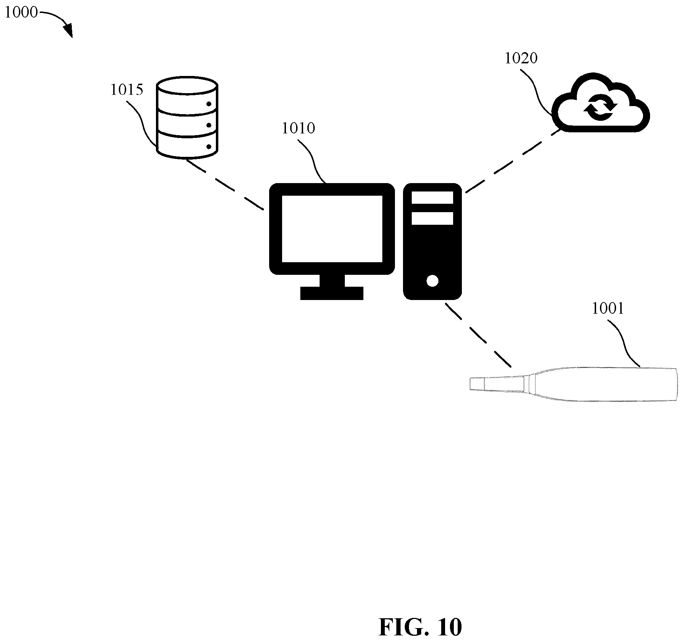

FIG. 10 illustrates an intraoral scanning system 1000 which may comprise a computer 1010 capable of carrying out any method of the disclosure. The computer may comprise a wired or a wireless interface to a server 1015, a cloud server 1020 and the intraoral scanner 1001. The intraoral scanner 1001 may be capable of recording the scan data comprising geometrical information, natural color information and/or fluorescence information associated with the patient's dentition. The intraoral scanner 1001 may be equipped with various modules such as a fluorescence module and/or an infrared module and thereby capable of capturing information relevant for diagnosing dental conditions such as caries, dental recession, tooth cracks, gingivitis, tartar, oral cancer and/or plaque.

The intraoral scanning system 1000 may comprise a data processing device configured to carry out the method according to one or more embodiments of the disclosure. The data processing device may be a part of the computer 1010, the server 1015 or the cloud server 1020.

A non-transitory computer-readable storage medium or a computer-readable storage medium may be comprised in the dental scanning system 1000. The non-transitory computer-readable medium can carry instructions which, when executed by a computer, cause the computer to carry out the method according to one or more embodiments of the disclosure.

The computer-readable storage medium may comprise an optical storage media such as an optical disc, a machine-readable bar code, a USB flash memory, a magnetic storage device, a solid-state electronic storage device such as random access memory (RAM), or read only memory (ROM), or any other physical device or medium employed to store a computer program. Thereby the embodiments of the disclosure can be utilized on a data processing hardware apparatus, such as a computer system or personal computer, or on an embedded system that employs a dedicated data processing unit, such as a digital signal processing chip.

A computer program product may be embodied in the non-transitory computer-readable storage medium. The computer program product may comprise instructions which, when executed by a computer, cause the computer to perform the method according to any of the embodiments presented herein.

References throughout this disclosure to certain features, advantages, or similar does not imply that all of the features and advantages of the present disclosure should be or are in any single embodiment. Instead, it is to be understood that a specific feature, or characteristic described in connection with an embodiment is included in at least one embodiment of the disclosure. Thus, discussion of the features and advantages, and similar language, throughout this disclosure may, but do not necessarily, refer to the same embodiment.

### List of items:

1. A computer-implemented method for determining a confidence score of a digital dental workflow, the method comprising:
   - receiving scan data representative of a dental situation;
   - determining a quality measure of the received scan data;
   - generating a digital 3D model based on the received scan data;
   - determining a quality measure of the generated digital 3D model;
   - identifying, on the digital 3D model and/or based on the scan data, a dental condition and determining a probability value that may represent a likelihood that the dental condition is identified correctly;
   - determining a confidence score of the digital dental workflow based on the quality measure of the received scan data, the quality measure of the generated digital 3D model and/or the probability value;
   - displaying the digital 3D model with the dental condition, and
   - displaying the confidence score.
2. The method according to item 1, wherein the scan data comprises geometric data, color images, fluorescence images and/or near-infrared images.
3. The method according to any previous item, wherein determining the quality measure of the received scan data comprises determining a signal-to-noise ratio of color images.
4. The method according to any previous item, wherein determining the quality measure of the received scan data comprises determining a signal-to-noise ratio of fluorescence images.
5. The method according to any previous item, wherein determining the quality measure of the received scan data comprises determining a signal-to-noise ratio of near-infrared images.
6. The method according to any previous item, wherein determining the quality measure of the received scan data comprises determining an overlap between images of different modalities.
7. The method according to the previous item 6, wherein determining an overlap between images of different modalities comprises geometrically comparing the color images, the fluorescence images and/or the near-infrared images.
8. The method according to the previous item 7, wherein geometrically comparing the color images, the fluorescence images and/or the near-infrared images comprises identifying a feature such as an edge of the tooth in the images to be compared, and testing whether the identified feature overlaps when the images are compared.
9. The method according to any previous item, wherein determining the quality measure of the received scan data comprises determining an amount of overlap of the digital 3D model and the fluorescence images overlaid onto the digital 3D model.
10. The method according to any previous item, wherein determining the quality measure of the received scan data comprises determining a noise level in a sub-scan, wherein the sub-scan represents a geometry of a part of the digital 3D model and wherein the sub-scan is generated from a series of color images of the scan data.
11. The method according to the previous item 10, wherein the noise level in the sub-scan is a level of surface roughness of the sub-scan.
12. The method according to the previous item 10, wherein the noise level in the sub-scan is a function of an amplitude of a correlation measure between a reference signal projected by an intraoral scanner onto an object being scanned, and a recorded light signal by the intraoral scanner.
13. The method according to any previous item, wherein determining the quality measure of the received scan data comprises mutually comparing color images of the sub-scan.
14. The method according to the previous item 13, wherein mutually comparing color images comprises comparing corresponding color intensity values of color images.
15. The method according to the previous item 14, wherein the color intensity values are weighted by a scan angle.
16. The method according to any previous item 13 or 14, further comprising determining a variation in the color intensity values of color images.
17. The method according to any previous item, wherein determining the quality measure of the received scan data comprises identifying a glare effect in at least one of the near-infrared images.
18. The method according to the previous item 17, wherein identifying the glare effect comprises comparing a contrast profile of a potential glare effect to a predetermined contrast profile of the glare effect.
19. The method according to any previous item, wherein determining the quality measure of the received scan data comprises determining an amount of glare effects in the near-infrared images.
20. The method according to any previous item, wherein determining the quality measure of the received scan data comprises determining an amount of glare effects in the near-infrared images per sub-scan.
21. The method according to any previous item, wherein determining the quality measure of the received scan data comprises detecting a removal of the glare effect in at least one of the near-infrared images.
22. The method according to the previous item 21, wherein the removal of the glare effect comprises combining near-infrared images of a region for which the removal of the glare effect is desired, wherein combining is performed for the near-infrared images of at least two sub-scans.
23. The method according to the previous item 22, wherein combining of the near-infrared images is performed for those near-infrared images for which the region with the glare effect is in focus.
24. The method according to any previous item, wherein determining the quality measure of the received scan data is performed in real-time during acquisition of the scan data.
25. The method according to any previous item 1-23, wherein determining the quality measure of the received scan data is performed in post-processing stage after generation of the digital 3D model.
26. The method according to any previous item, further comprising displaying the determined quality measure of the received scan data.
27. The method according to the previous item 26, wherein the quality measure of the received scan data is displayed as a color overlay on the digital 3D model.
28. The method according to the previous item 26 or 27, wherein the quality measure of the received scan data is displayed during generation of the digital 3D model.
29. The method according to any previous item, wherein determining the quality measure of the received scan data comprises identifying a part of the scan data representing a tooth and determining the quality measure of the part of the scan data representing the tooth.
30. The method according to the previous item 29, wherein identifying the part of the scan data representing the tooth comprises use of a trained neural network for segmenting the scan data.
31. The method according to the previous item 30, wherein the trained neural network for segmenting the scan data has been trained on training data comprising annotated near-infrared images, annotated color images and/or annotated fluorescence images.
32. The method according to the previous item 29 or 30, wherein the scan data comprises the near-infrared images and/or composed images derived from the near-infrared images.
33. The method according to the previous item 32, wherein the training data comprises annotated composed images, wherein the composed images are derived from the near-infrared images by combining the near-infrared images, the fluorescence images and/or the color images.
34. The method according to any previous item, wherein determining the quality measure of the received scan data is performed based on a series of images of the scan data acquired within a predetermined timeframe, wherein the predetermined timeframe is selected so that the acquired series of images of the scan data corresponds to a tooth.
35. The method according to the previous item 34, wherein the predetermined timeframe is between 1 second and 5 seconds.
36. The method according to the previous item 34 or 35, wherein the series of images of the scan data comprise a series of near-infrared images and/or a series of composed images, wherein the composed images are derived from the near-infrared images.
37. The method according to any previous item, wherein determining the quality measure of the received scan data is performed for a series of color images acquired during an acquisition cycle for the color images, wherein the acquisition cycle for the color images is in a range from 100 to 200 milliseconds.
38. The method according to any previous item, wherein determining the quality measure of the received scan data is performed for a series of fluorescence images acquired during an acquisition cycle for the fluorescence images.
39. The method according to any previous item, wherein determining the quality measure of the received scan data is performed for a series of near-infrared images acquired during an acquisition cycle for the near-infrared images.
40. The method according to any previous item, wherein determining the quality measure of the generated digital 3D model comprises determining a surface area of the generated digital 3D model for which color information is missing.
41. The method according to the previous item 40, further comprising comparing the determined surface area to a first surface area threshold.
42. The method according to the previous item 40, further comprising determining a ratio of the determined surface area of the generated digital 3D model for which color information is missing to the complete surface area of the generated digital 3D model.
43. The method according to any previous item, wherein determining the quality measure of the generated digital 3D model comprises determining a surface area of the generated digital 3D model for which fluorescence information is missing.
44. The method according to the previous item 43, further comprising comparing the determined surface area of the generated digital 3D model for which fluorescence information is missing to a second surface area threshold.
45. The method according to any previous item, further comprising displaying the quality measure of the generated digital 3D model on the digital 3D model.
46. The method according to any previous item, wherein identifying the dental condition comprises applying a trained neural network for identifying the dental condition to at least a part of the scan data.
47. The method according to any previous item, wherein the probability value representing the likelihood that the dental condition is identified correctly is provided as an output of the trained neural network for identifying the dental condition.
48. The method according to any previous item 1-47, wherein identifying the dental condition comprises comparing a contrast profile of a potential dental condition to a predetermined contrast profile of the dental condition.
49. The method according to the previous item 48, wherein the probability value is determined based on a fitting error obtained after fitting the contrast profile of the potential dental condition to the predetermined contrast profile of the dental condition.
50. The method according to the previous item 48 or 49, wherein the predetermined contrast profile of the dental condition is obtained by using a trained neural network trained on a plurality of contrast profiles of dental conditions.
51. The method according to any previous item, further comprising displaying the probability value.
52. The method according to any previous item, wherein identifying the dental condition comprises identifying a type of the dental condition, a severity of the dental condition, a volume of the dental condition and/or a surface area of the dental condition.
53. The method according to any previous item, further comprising applying a first weight coefficient to the quality measure of the received scan data.
54. The method according to any previous item, further comprising applying a second weight coefficient to the quality measure of the generated digital 3D model.
55. The method according to any previous item, further comprising applying a third weight coefficient to the probability value.
56. The method according to any previous item 53-55, wherein the first weight coefficient, the second weight coefficient and/or the third weight coefficient are derived empirically.
57. The method according to any previous item, further comprising detecting user input specifying a modified probability value and determining the confidence score of the digital dental workflow based on the modified probability value.
58. A computer-implemented method for determining a neural network confidence score, the method comprising:
   - receiving a digital 3D model representative of a dental situation;
   - identifying a dental condition on at least a part of the digital 3D model using a trained neural network for identifying the dental condition, wherein the trained neural network for identifying the dental condition has been trained on training data comprising a plurality of annotated training samples;
   - determining a neural network confidence score associated with the identified dental condition; and
   - displaying the neural network confidence score.
59. The method according to the previous item 50, wherein the neural network confidence score is determined based on analysing the training data.
60. The method according to the previous item 59, wherein analysing the training data comprises determining a distribution of the training data.
61. The method according to the previous item 60, wherein the distribution of the training data is a distribution of the training samples.
62. The method according to any previous item 58-61, further comprising determining an outlier in the training data.
63. The method according to the previous item 62, wherein the outlier is a training sample comprising retainers, brackets, a preparation for a restoration, a filling, a restoration, a third molar, and/or a number of teeth less than a threshold.
64. The method according to the previous item 62 or 63, further comprising generating a latent space representation of the outlier.
65. The method according to the previous item 64, further comprising storing the generated latent space representation in a list.
66. The method according to the previous item 64 or 65, further comprising generating the latent space representation of the digital 3D model that is input into the trained neural network.
67. The method according to the previous item 66, further comprising comparing the latent space representation of the digital 3D model to the latent space representation of the outlier to obtain a similarity measure.
68. The method according to the previous item 67, further comprising assigning the neural network confidence score based on the obtained similarity measure.
69. The method according to the previous item 68, further comprising:
   - applying the trained neural network for identifying the dental condition to a test data comprising a plurality of test samples;
   - identifying a test sample from the plurality of test samples satisfying a criterion;
   - determining the neural network confidence score based on the correspondence of the test sample and the digital 3D model that is input into the trained neural network for identifying the dental condition.
70. The method according to the previous item 69, wherein the criterion comprises determining that a percentage of the correctly classified area of the test sample is below a predetermined area threshold.
71. The method according to the previous item 70, further comprising storing the test sample in a confidence score list.
72. The method according to the previous item 71, further comprising generating a performance plot during application of the trained neural network to the test data and generating a latent space cluster for part of the test data for which the performance of the trained neural network is below a performance threshold.
73. The method according to the previous item 72, further comprising generating the latent space representation of the digital 3D model that is input into the trained neural network.
74. The method according to the previous item 73, further comprising comparing the latent space representation of the digital 3D model to the latent space cluster to obtain the similarity measure.
75. The method according to the previous item 74, further comprising assigning the neural network confidence score based on the obtained similarity measure.
76. An intraoral scanner system configured to determine a confidence score of a digital dental workflow, wherein the intraoral scanner system comprises:
   - an intraoral scanner configured to obtain scan data representative of a dental situation,
   - a display configured to display a digital 3D model of the dental situation,
   - a processor configured to:
   - receive scan data representative of the dental situation,
   - determine a quality measure of the received scan data,
   - generate the digital 3D model based on the received scan data,
   - determine a quality measure of the generated digital 3D model,
   - identify, on the digital 3D model and based on the scan data, a dental condition, and determine a probability value representing the likelihood that the dental condition is identified correctly,
   - determine a confidence score of the digital dental workflow based on the quality measure of the received scan data, the quality measure of the generated digital 3D model and/or the probability value,
   - display the digital 3D model with the dental condition, and
   - display the confidence score of the digital dental workflow.
77. An intraoral scanner system configured to determine a confidence score of a digital dental workflow, wherein the intraoral scanner system comprises:
   - an intraoral scanner configured to obtain scan data representative of a dental situation,
   - a display configured to display a digital 3D model of the dental situation,
   - a processor configured to:
   - receive scan data representative of the dental situation,
   - determine a quality measure of the received scan data,
   - generate the digital 3D model based on the received scan data,
   - determine a quality measure of the generated digital 3D model,
   - identify, on the digital 3D model and based on the scan data, a dental condition and determine the confidence score associated with the identified dental condition,
   - determine the confidence score of the digital dental workflow based on the quality measure of the received scan data, the quality measure of the generated digital 3D model and/or the confidence score associated with the identified dental condition,
   - display the digital 3D model with the dental condition, and
   - display the confidence score of the digital dental workflow.
78. An intraoral scanner system configured to determine a confidence score of a digital dental workflow, wherein the intraoral scanner system comprises:
   - an intraoral scanner configured to obtain scan data representative of a dental situation, wherein the scan data comprises color images and near-infrared images,
   - a display configured to display a digital 3D model of the dental situation,
   - a processor configured to:
   - receive scan data representative of the dental situation,
   - determine a quality measure of the received scan data,
   - generate enhanced diagnostic images based on at least the near-infrared images of the scan data,
   - determine quality measure of the enhanced diagnostic images,
   - identify a dental condition in the dental situation based on the enhanced diagnostic images,
   - determine a probability value representing a likelihood that the dental condition is identified correctly,
   - determine a confidence score of the digital dental workflow based on the quality measure of the received scan data, the quality measure of the enhanced diagnostic images and the probability value.
79. The intraoral scanner system according to the previous item 78, wherein the processor is further configured to:
   - generate the digital 3D model based on the color images of the scan data,
   - align the confidence score onto the digital 3D model, and
   - display the digital 3D model with the aligned confidence score.
80. The intraoral scanner system according to the previous item 79, wherein generating the digital 3D model of the dental situation based on the color images of the scan data and determining the quality measure of the received scan data are performed **in** real-time during receiving of the scan data, and wherein the processor is further configured to:
   - align the quality measure of the received scan data onto the digital 3D model;
   - display the aligned quality measure of the received scan data and the digital 3D model **in** real time during receiving of the scan data.
81. The intraoral scanner system according to any the previous item 78-80, wherein the processor is further configured to:
   - enable displaying of the quality measure of the received scan data, the quality measure of the enhanced diagnostic images and the confidence score alone or **in** combination, based on a user input.
82. A computer-implemented method for determining a confidence score of a digital dental workflow, the method comprising:
   - receiving scan data representative of a dental situation, wherein the scan data comprises color images and near-infrared images,
   - determining a quality measure of the received scan data,
   - generating enhanced diagnostic images based on the at least near-infrared images of the scan data,
   - determining a quality measure of the enhanced diagnostic images,
   - identifying a dental condition **in** the dental situation based on the enhanced diagnostic images,
   - determining a probability value representing a likelihood that the dental condition is identified correctly,
   - determining a confidence score of the digital dental workflow based on the quality measure of the received scan data, the quality measure of the enhanced diagnostic images and the probability value.
83. The method according to the previous item 82, further comprising displaying the confidence score of the digital dental workflow.
84. The method according to the previous item 82, further comprising:
   - generating a digital 3D model of the dental situation based on the color images of the scan data;
   - aligning the confidence score onto the digital 3D model;
   - displaying the digital 3D model with the aligned confidence score.
85. The method according to the previous item 84, wherein aligning the confidence score onto the digital 3D model is performed based on the geometric data.
86. The method according to any previous item 84-85, wherein generating the digital 3D model of the dental situation based on the color images of the scan data and determining the quality measure of the received scan data are performed in real-time during receiving of the scan data, further comprising:
   - aligning the quality measure of the received scan data onto the digital 3D model;
   - displaying the aligned quality measure of the received scan data and the digital 3D model in real time during receiving of the scan data.
87. The method according to any previous item, further enabling displaying of the quality measure of the received scan data, the quality measure of the enhanced diagnostic images, the quality measure of the generated digital 3D model and the confidence score alone or in combination, based on a user input.
88. The method according to any previous item 82-87, wherein determining the confidence score of the digital dental workflow comprises selecting a lowest value of the quality measure of the received scan data, the quality measure of the enhanced diagnostic images and the probability value.
89. The method according to any previous item 82-87, wherein determining the confidence score of the digital dental workflow comprises selecting a majority value out of the quality measure of the received scan data, the quality measure of the enhanced diagnostic images and the probability value.

## Claims

1. A computer-implemented method for determining a confidence score of a digital dental workflow, the method comprising:
- receiving scan data representative of a dental situation, wherein the scan data comprises color images and near-infrared images;
- determining a quality measure of the received scan data;
- generating enhanced diagnostic images based on at least the near-infrared images of the scan data;
- determining a quality measure of the enhanced diagnostic images;
- identifying a dental condition in the dental situation based on the enhanced diagnostic images;
- determining a probability value representing a likelihood that the dental condition is identified correctly;
- determining a confidence score of the digital dental workflow based on the quality measure of the received scan data, the quality measure of the enhanced diagnostic images and the probability value; and
- displaying the confidence score of the digital dental workflow.

2. The method according to the previous claim 1, further comprising:
- generating a digital 3D model of the dental situation based on the color images of the scan data;
- aligning the confidence score onto the digital 3D model;
- displaying the digital 3D model with the aligned confidence score.

3. The method according to the previous claim 2, wherein generating the digital 3D model of the dental situation based on the color images of the scan data and determining the quality measure of the received scan data are performed in real-time during receiving of the scan data, the method further comprising:
- aligning the quality measure of the received scan data onto the digital 3D model;
- displaying the aligned quality measure of the received scan data and the digital 3D model in real time during receiving of the scan data.

4. The method according to any previous claim, further displaying the quality measure of the received scan data, the quality measure of the enhanced diagnostic images, the probability value and the confidence score alone or in combination, based on a user input.

5. The method according to any previous claim, wherein determining the quality measure of the received scan data comprises determining a signal-to-noise ratio of the color images and determining a signal-to-noise ratio of the near-infrared images.

6. The method according to any previous claim, wherein determining the quality measure of the received scan data comprises determining an overlap between images of different modalities by geometrically comparing the color images and the near-infrared images.

7. The method according to any previous claim, wherein determining the quality measure of the received scan data comprises determining a noise level in a sub-scan, wherein the sub-scan represents a geometry of a part of the digital 3D model and wherein the sub-scan is generated from a series of color images of the scan data.

8. The method according to the previous claim 7, wherein the noise level in the sub-scan is a surface roughness of the sub-scan.

9. The method according to any previous claim, wherein generating enhanced diagnostic images based on at least the near-infrared images of the scan data comprises generating composed images by combining intensity levels of pixels of an image sensor in an intraoral scanner, the pixels being associated with obtaining the color images and the near-infrared images.

10. The method according to any previous claim 1-8, wherein generating enhanced diagnostic images based on at least the near-infrared images of the scan data comprises generating optimized near-infrared images by applying a contrast enhancement algorithm to the near-infrared images, wherein the contrast enhancement algorithm is a non-linear enhancement algorithm such as a histogram equalization or an adaptive histogram equalization.

11. The method according to any previous claim, wherein determining the quality measure of the enhanced diagnostic images comprises determining an amount of glare effects in the enhanced diagnostic images per sub-scan.

12. The method according to any previous claim, wherein identifying the dental condition in the dental situation based on the enhanced diagnostic images comprises comparing a contrast profile of a potential dental condition to a predetermined contrast profile of the dental condition, wherein the contrast profile of the potential dental condition is generated by plotting pixel values of those pixels of a near-infrared image or an enhanced diagnostic image representing part of the dentition with suspected dental condition and wherein the predetermined contrast profile of the dental condition is obtained by averaging a plurality of previously determined contrast profiles of the dental condition.

13. The method according to the previous claim 12, wherein the probability value is determined based on a fitting error obtained after fitting the contrast profile of the potential dental condition to the predetermined contrast profile of the dental condition.

14. The method according to any previous claim, further comprising displaying a lowest value of the quality measure of the received scan data, the quality measure of the enhanced diagnostic images and the probability value.

15. The method according to the previous claim 14, further comprising generating a recommendation for a corrective action based on the lowest value.
